(19) Europäisches Patentamt
European Patent Office
Office européen des brevets

(11) Veröffentlichungsnummer: **0 272 573**
**B1**

(12) # EUROPÄISCHE PATENTSCHRIFT

(45) Veröffentlichungstag der Patentschrift: **06.06.90**

(51) Int. Cl.⁵: **G03C 7/32**
// C07D249/04

(21) Anmeldenummer: **87118470.1**

(22) Anmeldetag: **14.12.87**

(54) **Farbfotografisches Aufzeichnungsmaterial mit einem Kuppler, der eine fotographisch wirksame Verbindung freisetzt.**

(30) Priorität: **24.12.86 DE 3644416**
**24.12.86 DE 3644405**

(43) Veröffentlichungstag der Anmeldung:
**29.06.88 Patentblatt 88/26**

(45) Bekanntmachung des Hinweises auf die Patenterteilung:
**06.06.90 Patentblatt 90/23**

(84) Benannte Vertragsstaaten:
**BE DE FR GB NL**

(56) Entgegenhaltungen:
**FR-A- 2 232 780**
**FR-A- 2 334 982**
**US-A- 4 049 458**

(73) Patentinhaber: **Agfa-Gevaert AG,**
**D-5090 Leverkusen 1(DE)**

(72) Erfinder: **Odenwälder, Heinrich, Dr., Am Arenzberg 37,**
**D-5090 Leverkusen 3(DE)**
Erfinder: **Vetter, Hans, Dr., Gerstenkamp 19,**
**D-5000 Köln 80(DE)**
Erfinder: **Bergthaller, Peter, Dr., Leuchter Gemark 5A,**
**D-5060 Bergisch Gladbach 2(DE)**
Erfinder: **Hübner, Dirk, Dr.,**
**Andreas-Gryphius-Strasse 11, D-5000 Köln 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 272 573 B1

**Beschreibung**

Die Erfindung betrifft ein farbfotografisches Aufzeichnungsmaterial mit mindestens einer lichtempfindlichen Silberhalogenidemulsionsschicht, die einen Kuppler enthält, der bei Farbentwicklung eine fotografisch wirksame Gruppe, z.B. einen Entwicklungsinhibitor freisetzt.

Es ist bekannt, die chromogene Entwicklung in Gegenwart von Verbindungen durchzuführen, die bei der Entwicklung bildmäßig diffusionsfähige Substanzen freisetzen, die eine bestimmte Wirkung entfalten, beispielsweise die Entwicklung von Silberhalogenid zu beeinflussen vermögen. Falls diese Wirkung darin besteht, daß die weitere Entwicklung inhibiert wird, werden derartige Verbindungen als sogenannte DIR-Verbindungen (DIR = development inhibitor releasing) bezeichnet. Bei den DIR-Verbindungen kann es sich um solche handeln, die unter Abspaltung eines Inhibitor restes mit dem Oxidationsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet.

DIR-Kuppler sind beispielsweise bekannt aus US-A-3 148 062, US-A-3 227 554, US-A-3 615 506, US-A-3 617 291 und DE-A-2 414 006.

Bei den diffusionsfähigen, fotografisch wirksamen Verbindungen, die bei der Entwicklung freigesetzt werden, kann es sich aber beispielsweise auch um einen Farbstoff, einen Kuppler, ein Härtungsmittel, ein Silberhalogenidlösungsmittel, ein Verschleierungsmittel, einen Entwicklungsbeschleuniger, eine Entwicklerverbindung, einen Bleichinhibitor, einen Bleichbeschleuniger, ein Beizmittel oder einen Sensibilisator handeln.

Bei freigesetzten Entwicklunsinhibitoren handelt es sich in der Regel um heterocyclische Mercaptoverbindungen oder um Derivate des Benzotriazols. Hinsichtlich der im wesentlichen farblos kuppelnden DIR-Verbindungen sei beispielsweise verwiesen auf US-A-3 632 345, DE-A-23 59 295 und DE-A-25 40 959. Durch Anwendung von DIR-Verbindungen kann eine Vielzahl von fotografischen, die Bildqualität beeinflussenden Effekten bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation "Development-Inhibitor-Releasing (DIR) Couplers of Color Photography" von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, daß sie universell einsetzbar sind, so daß die gleiche Verbindung ohne Rücksicht auf die zu erzeugende Farbe in allen lichtempfindlichen Schichten eines farbfotografischen Aufzeichnungsmaterials verwendet werden kann. DIR-Kuppler können dagegen wegen der aus ihnen erzeugten Farbe meist nur in einem Teil der lichtempfindlichen Schichten verwendet werden, falls nicht die auf sie zurückzuführende Farbnebendichte in den anderen Schichten tolerierbar ist. Diesem Vorteil der DIR-Verbindungen steht als Nachteil gegenüber, daß sie im allgemeinen weniger reaktive sind als die DIR-Kuppler. In der Praxis hat man sich daher meist darauf beschränkt, DIR-Kuppler zu verwenden und zwar notfalls zwei oder mehrere verschiedene im gleichen Aufzeichnungsmaterial, wobei den unterschiedlich spektral sensibilisierten Schichten verschiedene DIR-Kuppler nach Maßgabe der aus den letzteren erzeugten Farbe zuzuordnen waren.

Es ist normalerweise wichtig, daè die fotografisch wirksame Verbindung bei Entwicklung rasch aus dem Kuppler freigesetzt wird, und zwar insbesondere dann, wenn die fotografisch wirksame Verbindung den weiteren Verlauf der Entwicklung beeinflussen soll. Es ist daher sehr erwünscht, wenn die betreffenden Kuppler sehr aktiv sind. Hierbei kommt der an die Kupplungsstelle des Kupplers gebundene Gruppe der fotografisch wirksamen Verbindung als sogenannte Fluchtgruppe besondere Bedeutung zu.

In DE-A-28 42 063 sind DIR-Kuppler beschrieben, die sich von Gelbkupplern ableiten und als abspaltbaren Inhibitorrest einen 3-Alkylthio-1,2,4-triazolylrest enthalten. Bei Verwendung der dort beschriebenen DIR-Kuppler in einer blauempfindlichen Silberhalogenidemulsionsschicht kann zwar die Farbgradation in dieser Schicht beträchtlich verringert werden, jedoch ist die Auswirkung auf benachbarte Silberhalogenidschichten, insbesondere auf eine benachbarte grün- und/oder rotempfindliche Silberhalogenidemulsionsschicht vergleichsweise gering. Mit den bekannten DIR-Kupplern lassen sich daher nur geringe Interimageeffekte erzeugen.

Weiterhin sind in DE-A-34 27 235 DIR-Kuppler beschrieben, die sich ebenfalls von Gelbkupplern ableiten und die einen abspaltbaren 3-Alkylthio-5-furyl-1,2,4-triazolrest enthalten. Diese Kuppler zeigen eine zufriedenstellende Fernwirkung im Sinne eines Interimageeffektes, wenn sie in der blauempfindlichen Schicht eingesetzt werden. Wie können auch erfolgreich in grünempfindlichen Schichten eingesetzt werden; jedoch ist dann eine höhere Konzentration erforderlich um einen ausreichenden Interimageeffekt zu bewirken, wodurch eine zu hohe zu kompensierende gelbe Nebendichte entsteht. In rotempfindlichen Schichten sind diese Verbindungen nahezu wirkungslos.

Weiter ist in DE-A-26 55 871 ein Malonamidderivat beschrieben, das in der Kupplungsstelle einen in bestimmter Weise substiutierten 1,2,4-Triazolring trägt; die DIR-Kuppler-Wirkung dieser Verbindung ist aber ziemlich gering.

In US-A-4 049 458 sind verschiedene 2-Äquivalent-Kuppler beschrieben, die an die Kupplungsstelle gebunden einen 1,2,3-Triazolring enthalten. Der genannten Druckschrift ist nicht zu entnehmen, daß diese 1,2,3-Triazolgruppe nach Freisetzung eine bestimmte Wirkung entfaltet und tatsächlich konnte auch keine besondere Wirkung dieser Verbindungen festgestellt werden.

Der Erfindung liegt die Aufgabe zugrunde, ein farbfotografisches Aufzeichnungsmaterial anzugeben, das Kuppler mit einem an die Kupplungsstelle gebundenen 1,2,3-Triazolring enthält, aus denen bei der Entwicklung der 1,2,3-Triazolring als fotografisch wirksame Verbindung freigesetzt wird.

Gegenstand der Erfindung ist ein farbfotografisches Aufzeichnungsmaterial mit mindestens einer lichtempfindlichen Silberhalogenidemulsionsschicht und einem dieser zugeordneten Kuppler, der an seine Kupplungsstelle gebunden einen abspaltbaren 1,2,3-Triazolylrest trägt, dadurch gekennzeichnet, daß der Kuppler der folgenden Formel I entspricht

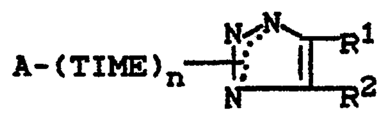

I

worin bedeuten

A den Rest eines Kupplers, der unter den Bedingungen der fotografischen Entwicklung mit dem Oxidationsprodukt eines Silberhalogenidentwicklungsmittels kuppelt und dabei den Rest der Formel

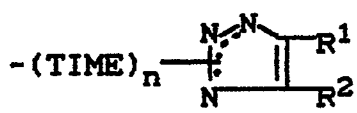

freisetzt;

TIME ein Bindeglied, das bei Reaktion des Kupplers mit dem Oxidationsprodukt eines Silberhalogenidentwicklungsmittels zusammen mit dem daran gebundenen Triazolring freigesetzt wird und seinerseits unter den Entwicklungsbedingungen den Triazolring verzögert freisetzt;

n 0, 1 oder 2;

$R^1$ H, Alkyl, Aryl, $-S-R^3$, $-O-R^3$, $-CO-R^3$, eine Carbonsäureestergruppe, eine gegebenenfalls substituierte Aminogruppe, $-CONR^4-R^5$ oder eine heterocyclische Gruppe;

$R^2$ Halogen, -OH, Alkyl, eine Phenylgruppe, die substituiert ist mit mindestens einem der folgenden Reste:

Halogen wie F, Cl, Br, I; Alkyl wie Methyl, Ethyl, Butyl; Aryl z.B. Phenyl; eine gegebenenfalls substituierte Aminogruppe, Alkoxy wie z.B. Methoxy; Aryloxy wie z.B. Phenoxy; Alkylthio wie z.B. Methylthio; Arylthio wie z.B. Phenylthio; Nitro; Cyano; $-CF_3$; Acyl wie z.B. Formyl oder Acetyl;

Naphtyl, $-S-R^3$, $-CO-R^3$, eine Carbonsäureestergruppe mit mindestens 3 C-Atomen, eine gegebenenfalls substituierte Aminogruppe, $-CONR^4-R^5$, Cyano oder eine heterocyclische Gruppe:

$R^3$ Alkyl, Cycloalkyl, Aralkyl, Aryl, Alkenyl oder Alkinyl;

$R^4$ Alkyl, Aralkyl, Aryl, Acyl, $-NH_2$ oder Acylamino:

$R^5$ Wasserstoff, einen Rest wie $R^4$

oder $R^4$ und $R^5$ zusammen den erforderlichen Rest zur Vervollständigung einer cyclischen Aminogruppe.

Der in Formel I durch A dargestellte Rest eines Kupplers kann der Rest eines Kupplers sein, der bei Farbentwicklung einen blaugrünen, purpurfarbenen oder gelben Farbstoff ergibt, oder auch der Rest eines Kupplers, der im wesentlichen farblose oder nur schwach farbige Produkte ergibt. Es handelt sich dabei im wesentlichen um bekannte Kupplerreste. Farbkuppler sind in großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen. Blaugrünkuppler weisen im allgmeinen phenolische oder naphtholische Struktur auf. Beispiele hierfür sind etwa beschrieben in US-A-2 369 929, US-A-2 772 162, EP-A-0 067 689, GB-A-519 208. Purpurkuppler leiten sich ab von 5-Pyrazolon oder verschiedenen Pyrazoloazolen. Beispiele sind etwa beschrieben in DE-A-25 36 191, DE-A-27 03 589 und DE-A-28 13 522, GB-A-1 247 493.

Gelbkuppler leiten sich beispielsweise ab von α-Acylacetaniliden wie Pivaloyl- oder Benzoylacetaniliden, oder Malonamiden. Beispiele sind etwa beschriben in US-A-2 875 057, US-A-3 265 506, US-A-4 359 521, DE-A-26 55 871. Kuppler, die im wesentlichen farblose Produkte liefern und gleichzeitig eine fotografisch wirksame Verbindung freisetzen, sind beispielsweise beschrieben in US-A-3 632 345, US-A-3 928 041, US-A-3 958 993, US-A-3 961 659, US-A-4 052 213, US-A-4 088 491.

In einer bevorzugten Ausführungsform ist der erfindungsgemäße eine fotografisch wirksame Verbindung freisetzende Kuppler ein DIR-Kuppler der folgenden Formel Ia

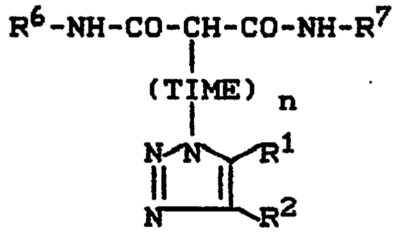

(Ia)

worin TIME, n, $R^1$ und $R^2$ die angegebene Beduetung haben und
worin bedeuten
$R^6$ eine heterocyclische oder carbocyclische aromatische Gruppe;
$R^7$ Alkyl oder einen Rest wie $R^6$.

Ein in Formel Ia durch $R^6$ oder $R^7$ dargestellter aromatischer Rest kann eine Arylgruppe sein, z.B> Phenyl oder eine heterocyclische Gruppe, z.B. Thiazol, Benzothiazol, Thienyl oder Pyridyl. Die genannten Gruppen können substituiert sein, z.B. durch Alkyl, Alkoxy, Halogen, Alkoxycarbonyl, Carbamoyl, Sulfamoyl oder Acylamino. Es ist bevorzugt, weil einer der Reste $R^6$ und $R^7$ oder beide Phenyl bedeuten, wobei im letzteren Fall beide Phenylreste unterschiedlich substituiert sein können.

Ein in Formel I oder Ia durch TIME dargestelltes Bindeglied ist eine Gruppe, die nach Abspaltung aus der Kupplungsstelle des Kupplers bei dessen Kupplung mit dem Oxidationsprodukt des Silberhalogenidentwicklungsmittels befähigt ist, in einer Folgereaktion einen daran gebundenen fotografisch wirksamen Rest, im vorliegenden Fall ein Triazol der Formel II freizusetzen. Die Gruppe TIME wird auch als Zeitsteuerglied bezeichnet, weil bei Anwesenheit einer solchen Gruppe der daran gebundene fotografisch wirksame Rest in vielen Fällen verzögert freigesestzt wird und wirksam werden kann. Bekannte Zeitsteuerglieder sind beispielsweise eine Gruppe

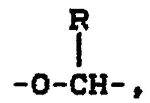

wobei das O-Atom an die Kupplungsstelle des Kupplers und das C-Atom an ein N-Atom einer fotografisch wirksamen Verbindung gebunden ist (z.B. DE-A-27 03 145), eine Gruppe, die nach Abspaltung vom Kuppler einer intramolekularen nukleophilen Verdrängungsreaktion unterliegt und hierbei die fotografisch wirksame Verbindung freisetzt (z.B. DE-A-28 55 697), eine Gruppe, in der nach Abspaltung vom Kuppler eine Elektronenübertragung entlang eines konjugierten Systems stattfinden kann, wodurch die fotografisch wirksame Verbindung freigesetzt wird (z.B. DE-A-31 05 026), oder eine Gruppe

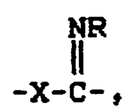

worin X (z.B. -O-) an die Kupplungsstelle des Kupplers und das C-Atom an ein Atom der fotografisch wirksamen Verbindung gebunden ist und worin R beispielsweise für Aryl steht (z.B. EP-A-0 127 063).

Die Gruppe TIME kann einfach, gegebenenfalls auch zweifach in gleicher oder verschiedener Strukturierung, vorhanden sein oder auch (im Fall n = 0) völlig fehlen.

Ein in Formel I oder Ia durch $R^1$, $R^2$, $R^3$, $R^4$ oder $R^7$ dargestellter oder in diesen Substituenten vorhandener Alkylrest kann geradkettig oder verzweigt, substituiert oder unsubstituiert sein und bis zu 20 C-Atomen enthalten; Beispiele sind Methyl, Ethyl, Propyl, Isopropyl, Butyl, s-Butyl, t-Butyl, Pentyl, Hexyl, Octyl, Dodecyl. Die Alkylreste können substituiert sein, z.B. durch Hydroxyl, Halogen, Alkoxy, Alkylthio, Alkenylthio, Acylamino oder eine cyclische Imidogruppe. Ein durch $R^1$, $R^2$ dargestellter Alkylrest oder ein beispielsweise an einen durch $R^1$ oder $R^2$ dargestellten Phenylrest als Substituent vorhandener Alkylrest enthält bevorzugt bis zu 4 C-Atome und ist insbesondere eine Methylgruppe.

Ein durch $R^3$ dargestellter Cycloalkylrest ist beispielsweise Cyclohexyl; ein Aralkylrest ($R^3$, $R^4$) ist beispielsweise Benzyl; ein Alkenylrest ist beispielsweise Allyl oder 2-Butenyl; ein Alkinylrest ist beispielsweise Propinyl.

Eine durch $R^1$ oder $R^2$ dargestellte gegebenenfalls substituierte Aminogruppe ist eine Aminogruppe, die ein- oder zweifach substituiert sein kann, z.B. durch Alkyl, Aralkyl, Aryl, Amino oder Acyl, und schließt cyclische Aminogruppen ein.

Eine z.B. durch $R^4$ und $R^5$ vervollständigte cyclische Aminogruppe ist beispielsweise eine Piperidino-, Morpholino-, Pyrrolino- oder Pyrazolylgruppe.

Ein Acylrest der beispielsweise in einem der durch $R^2$ und $R^4$ dargestellten Reste, z.B. in einer Acylaminogruppe, vorhanden sein kann, leitet sich ab von einer aliphatischen oder aromatischen Carbonsäure oder Sulfonsäure, einer Carbaminsäure, einer Sulfaminsäure oder einem Kohlensäuremonoester. Eine durch $R^1$ oder $R^2$ dargestellte Acylaminogruppe schließt beispielsweise die folgenen Gruppen ein: Ureido, Thioureido, Alkoxycarbonylamino und Thioacylamino.

Eine cyclische Imidogruppe ist beispielsweise eine Succinimidogruppe, Maleinimidogruppe, Phthalimidogruppe, Hexahydrophthalimidogruppe oder eine Gruppe der Formel

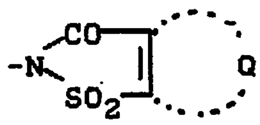

worin

Q den zur Vervollständigung eines carbocyclischen oder heterocyclischen, gegebenenfalls substituierten Ringes erforderlichen Rest bedeutet.

Eine durch $R^1$ oder $R^2$ dargestellte heterocyclische Gruppe ist beispielsweise eine Furyl-, Thiazolyl oder 1,2,4-Triazolylgruppe. Eine solche heterocyclische Gruppe kann weitere Substitenten tragen, z.B. Alkyl, Alkoxy, Acyl, Alkylthio ($-S-R^3$).

Die vorteilhaften Eigenschaften der erfindungsgemäßen Kuppler sind vermutlich darauf zurückzuführen, daß der 1,2,3-Triazolring offenbar nicht nur eine gute Abgangsgruppe (Fluchtgruppe) ist, so daß die Kuppler sehr reaktiv sind, sondern offenbar auch eine gewisse Neigung hat sich am Silberhalogenidkorn zu adsorbieren und hierbei die sich bei der Entwicklung des Silberhalogenids abspielenden Vorgänge zu beeinflussen. Die die Wirksamkeit der fotografisch wirksamen Verbindung bestimmenden Gruppen kommen hierbei offenbar in besonders günstigen Kontakt mit der Oberfläche der Silberhalogenidkorns. Es ist daher erfindungsgemäß bevorzugt, wenn die fotografisch wirksame Verbindung eine solche ist, die die Entwicklung des Silberhalogenids beeinflußt, z.B. ein Entwicklungsbeschleuniger, ein Verschleierungsmittel, ein Bleichbeschleuniger oder besonders bevorzugt ein Entwicklungsinhibitor. Namentlich im letzteren Fall ist es dabei weiter bevorzugt, wenn von den Resten $R^1$ und $R^2$ in den Formeln I und Ia mindestens einer für $-S-R^3$, $-COOR^8$ oder eine heterocyclische Gruppe steht, wobei $R^3$ für Alkyl mit vorzugsweise bis zu 7 C-Atomen, Cycloalkyl, Aralkyl, Alkenyl oder Alkinyl, und $R^8$ für Alkyl mit 2 bis 10 C-Atomen oder Aryl steht.

Bevorzugt werden Kuppler einer der beiden Formeln III und IV verwendet

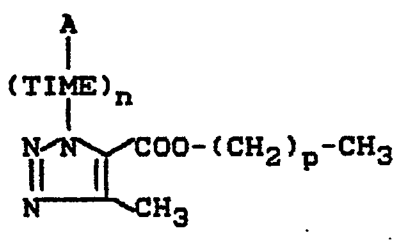

III

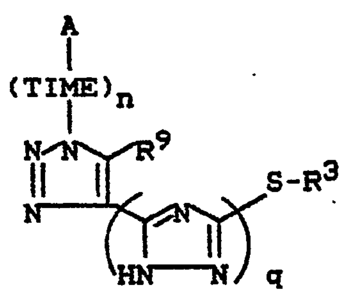

IV

worin

TIME die bereits angegebene Beduetung hat und worin bedeuten:

A den Rest eines Blaugrünkupplers eines Purpurkupplers, eines Gelbkupplers, insbesondere eines von Malonamid abgeleiteten Gelbkupplers, oder eines im wesentlichen farblos kuppelnden Kupplers;

$R^3$ Alkyl mit 1 bis 7 C-Atomen;

$R^9$ H, $-CH_3$, $-COO-(CH_2)_p-CH_3$;

n 0 oder 1;

p eine ganze Zahl von 1 bis 8;

q 0 oder 1.

In den Formeln III und IV ist jeweils nur eins von mehreren möglichen Isomeren hinsichtlich der Anknüpfung des Triazolringes an die Kupplungsstelle des Kupplers dargestellt. Die Formeln III und IV sollen sich aber gleichermaßen auch auf die anderen (hier nicht gezeigten) Isomeren beziehen.

Beispiele für fotografisch wirksame Verbindungen nach vorliegender Erfindung (vorwiegend Inhibitoren) sind im folgenden angegeben:

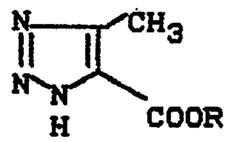

| INH- | R | |
|---|---|---|
| 1 | $-(CH_2)_p-CH_3$ | p = 3 |
| 2 | " | p = 4 |
| 3 | " | p = 5 |
| 4 | " | p = 6 |
| 5 | " | p = 7 |
| 6 | " | p = 8 |

| INH- | R |
|---|---|
| 7 | $-CH_2-C(CH_3)_3$ |
| 8 | 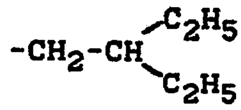 |
| 9 | 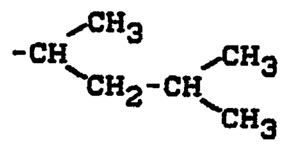 |
| 10 | $-CH_2-CF_2-CF_2-CF_3$ |
| 11 | $-(CH_2)_2-S-C_4H_9$ |
| 12 | $-(CH_2)_2-S-CH_2-CH=CH_2$ |
| 13 | 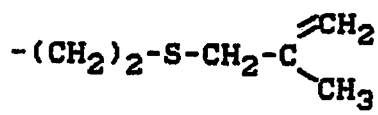 |
| 14 | $-(CH_2)_2-S-C_2H_5$ |
| 15 | 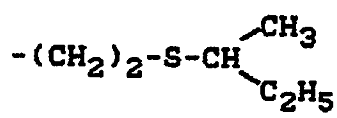 |
| 16 | $-(CH_2)_3-S-C_2H_5$ |
| 17 | $-(CH_2)_3-S-C_3H_7$ |
| 18 | $-(CH_2)_3-S-C_4H_9$ |
| 19 | 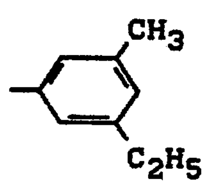 |
| 20 | 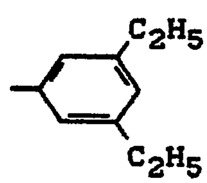 |

| INH- | R |
|---|---|
| 21 | 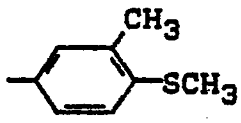 |
| 22 | 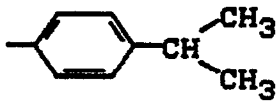 |
| 23 | 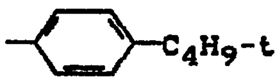 |
| 24 | 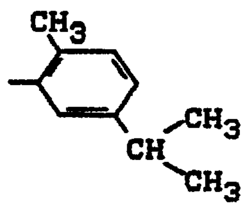 |
| 25 | 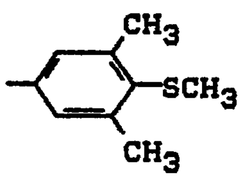 |
| 26 | 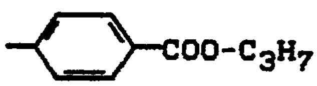 |
| 27 | 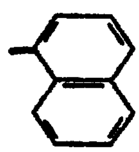 |
| 28 | 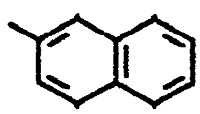 |

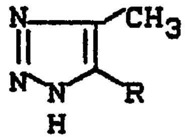

| INH- | R | |
|---|---|---|
| 29 | 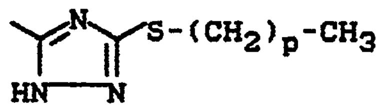 | p = 4 |
| 30 | " | p = 5 |
| 31 | " | p = 6 |
| 32 | " | p = 7 |

INH-33 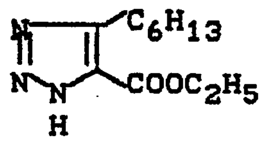

INH-34 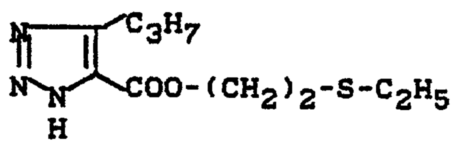

INH-35 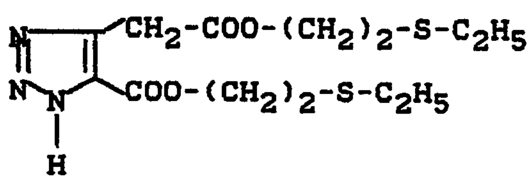

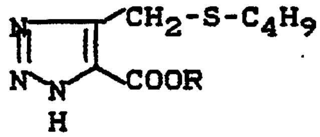

| INH- | R |
|---|---|
| 36 | $-C_2H_5$ |
| 37 | 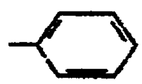 |
| 38 | 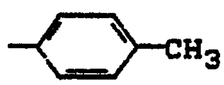 |
| 39 | 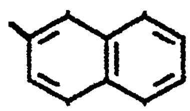 |

INH-40

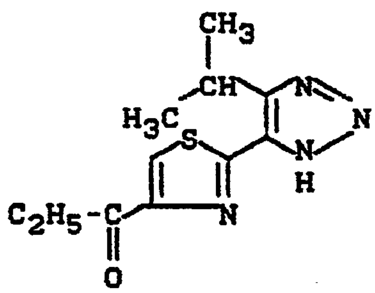

INH-41

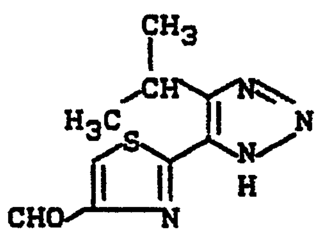

INH-42

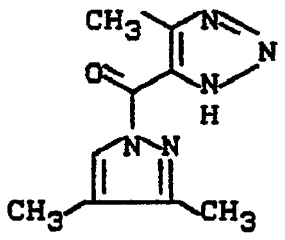

INH-43

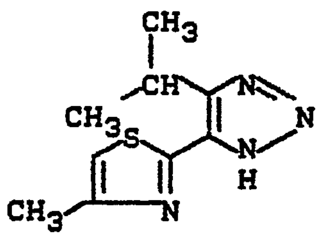

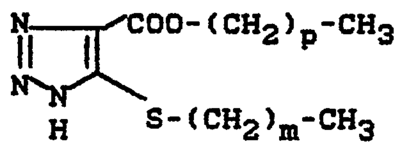

| INH- | p | m |
|---|---|---|
| 44 | 0 | 5 |
| 45 | 0 | 6 |
| 46 | 1 | 4 |
| 47 | 1 | 5 |
| 48 | 4 | 1 |
| 49 | 5 | 1 |

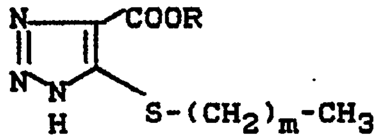

| INH- | R | m |
|---|---|---|
| 50 | 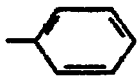 | 2 |
| 51 | 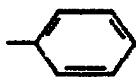 | 3 |
| 52 | 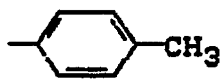 | 2 |
| 53 | 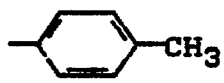 | 3 |
| 54 | 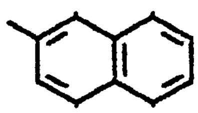 | 2 |
| 55 | 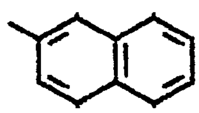 | 3 |

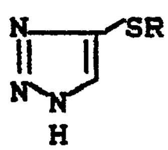

| INH- | R |
|---|---|
| 56 | $-C_4H_9$ |
| 57 | $-C_5H_{11}$ |
| 58 | 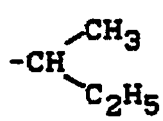 |
| 59 | 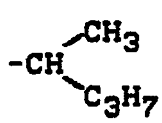 |

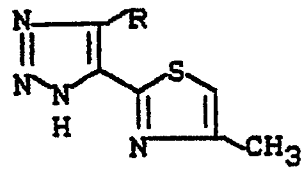

| INH- | R |
|---|---|
| 60 | 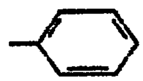 |
| 61 | 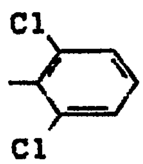 |
| 62 | 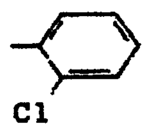 |
| 63 | $-S-C_4H_9$ |
| 64 | 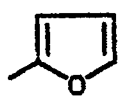 |

INH-65 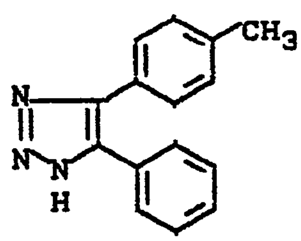

INH-66 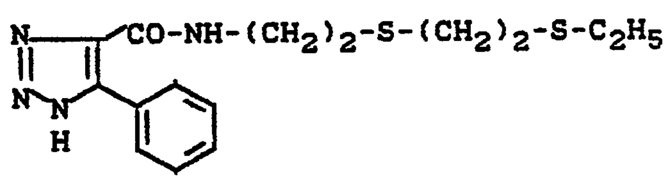

INH-67 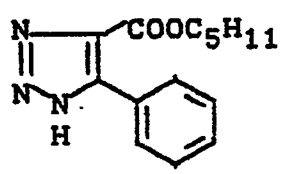

Beispiele für erfindungsgemäße Kuppler sind im folgenden angegeben:

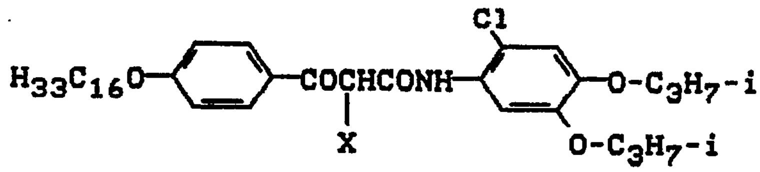

X =

K-1

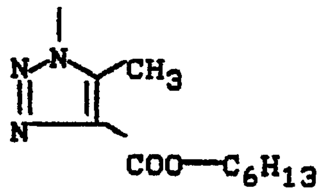

K-2 " $—C_4H_9$

K-3

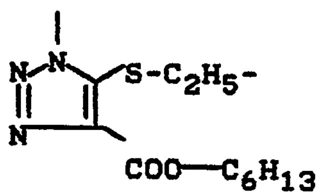

K-4

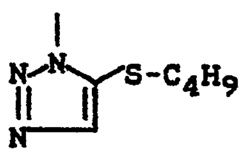

K-5

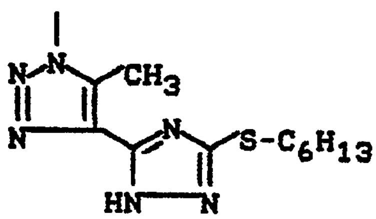

K-6

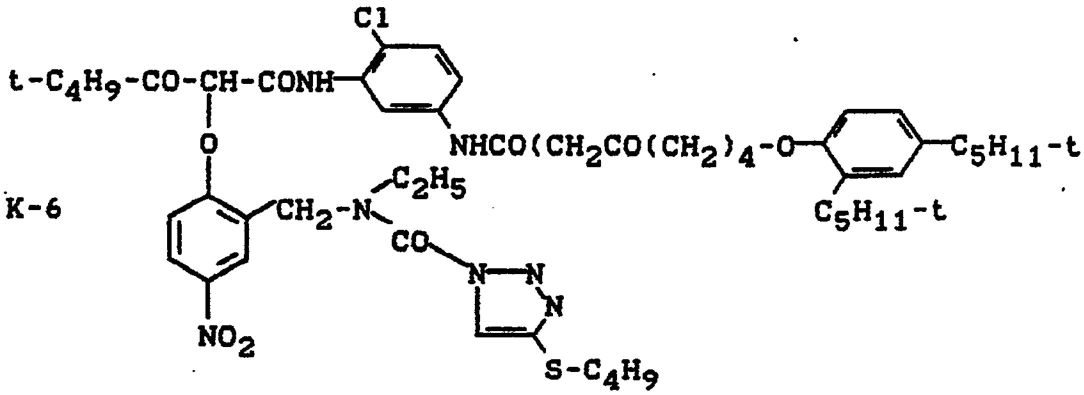

K-7

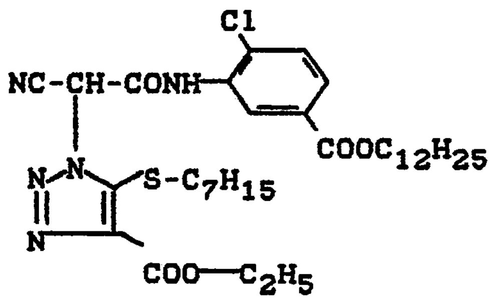

K-8

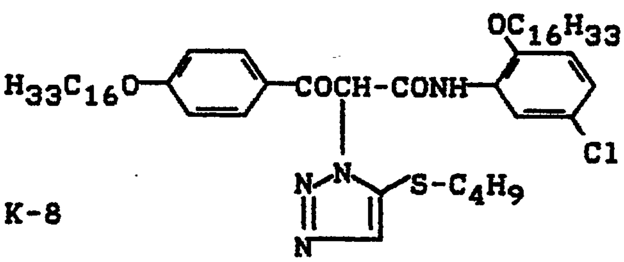

K-9

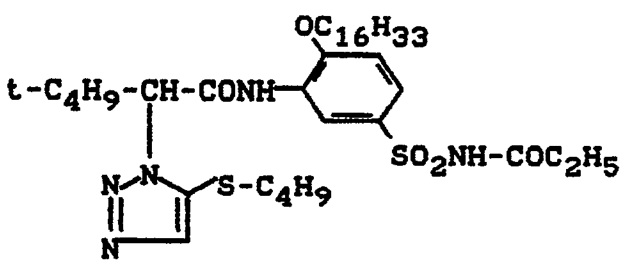

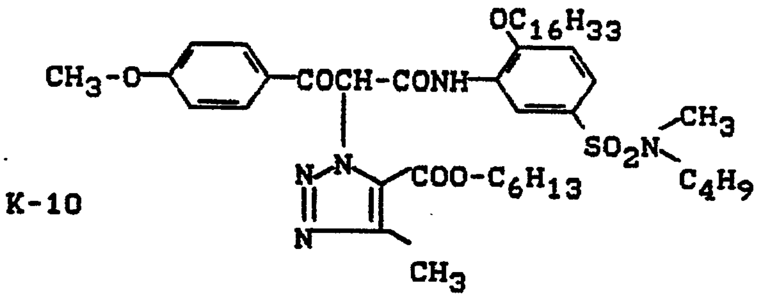

K-10

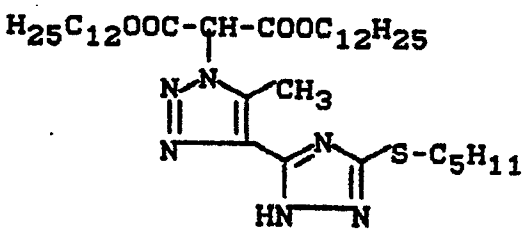

K-11

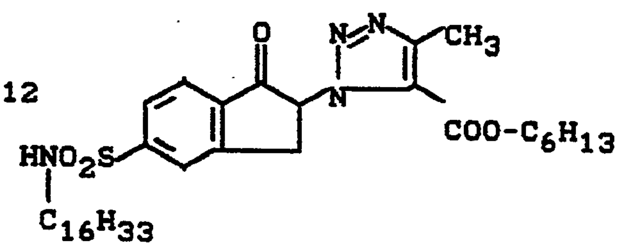

K-12

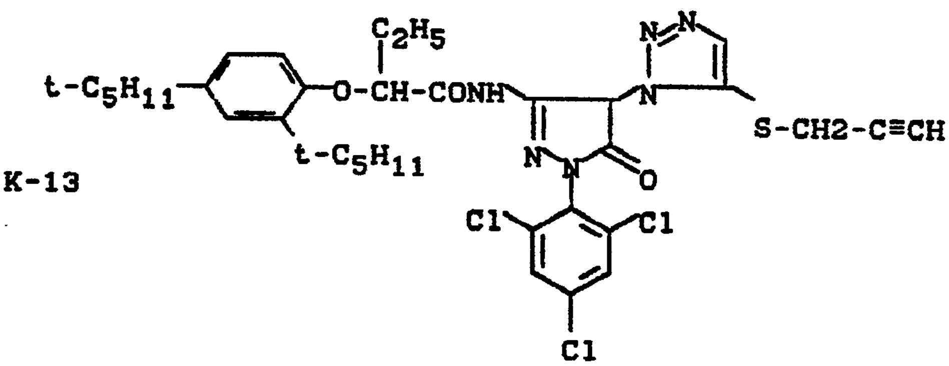

K-13

K-14

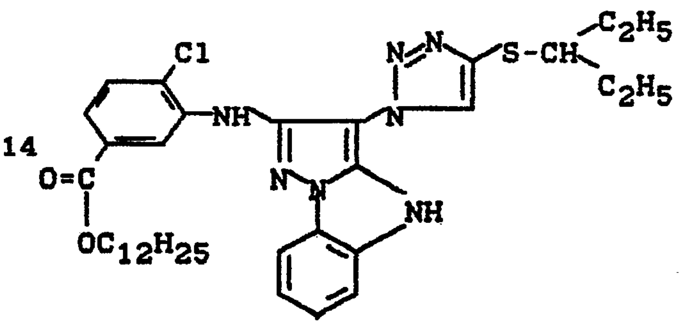

K-15

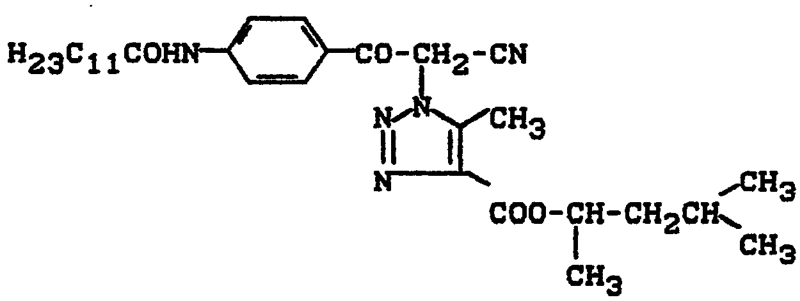

K-16

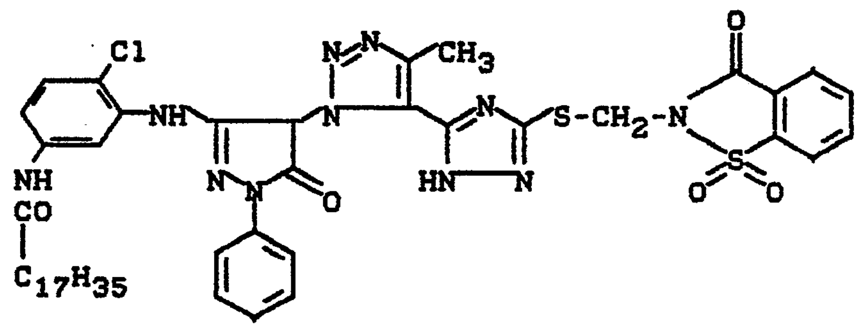

K-17

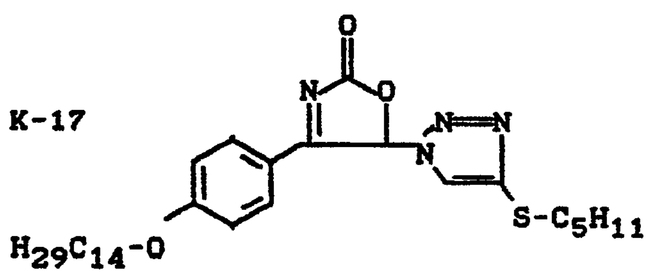

K-18

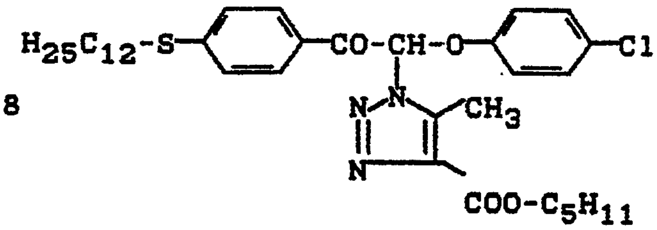

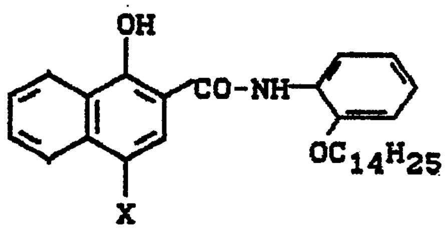

K-19 X = -O-$CH_2$-

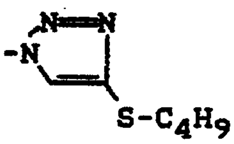

K-20 X = -

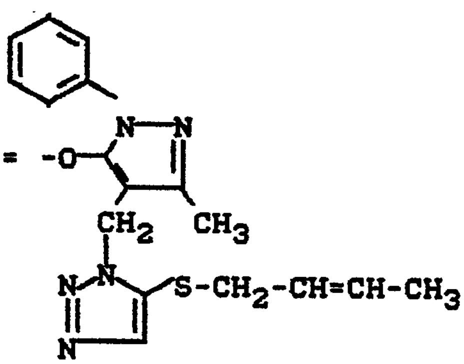

K-21 X = -

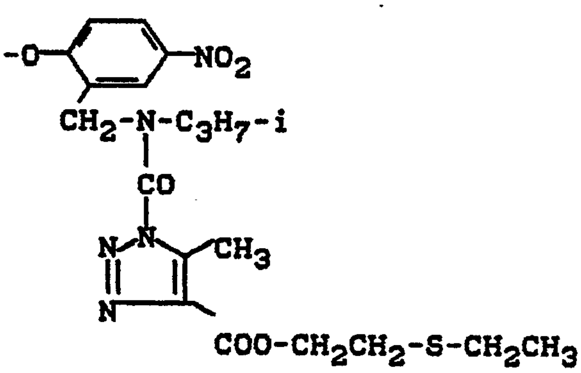

K-22 X =

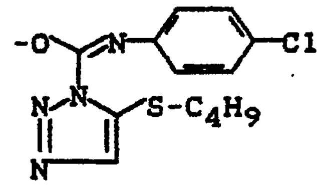

K-23

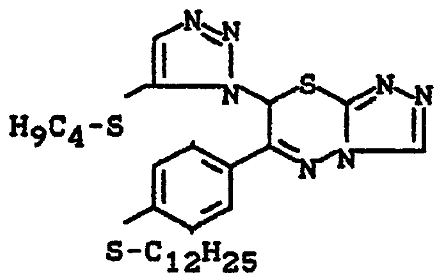

K-24

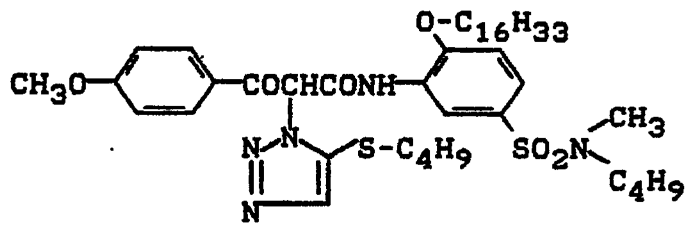

K-25

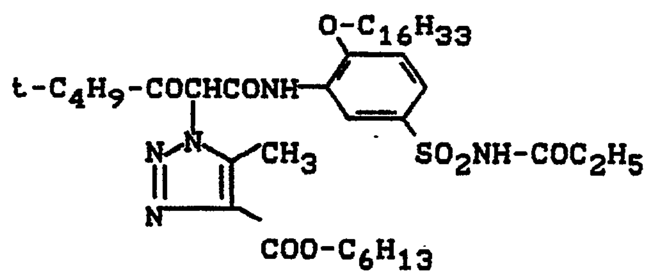

K-26

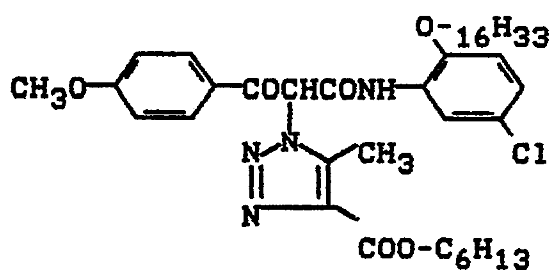

K-27

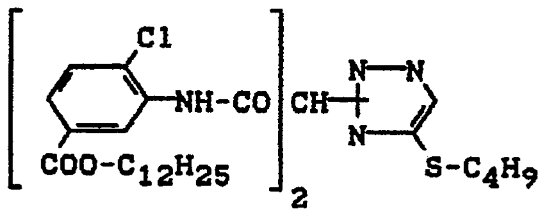

K-28

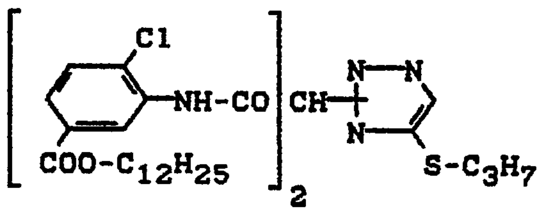

K-29

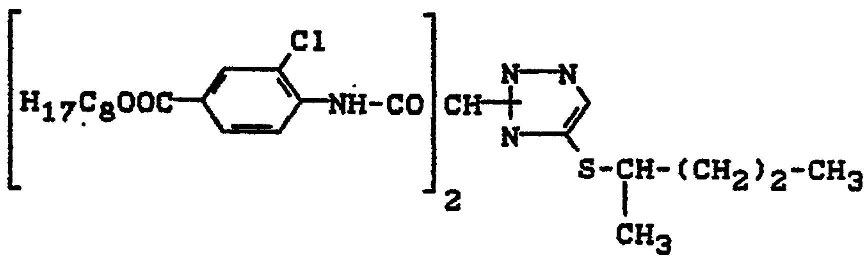

K-30

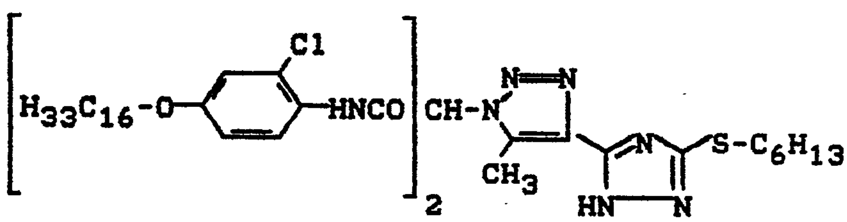

K-31

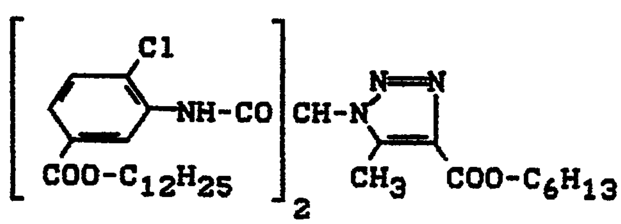

K-32

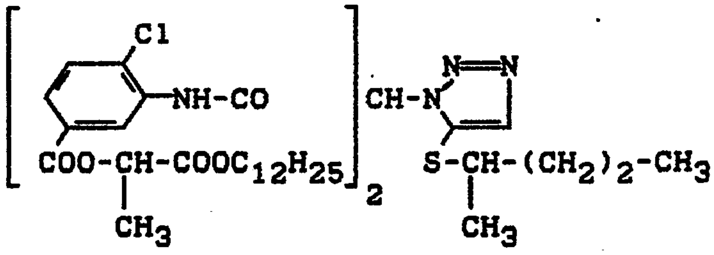

K-33

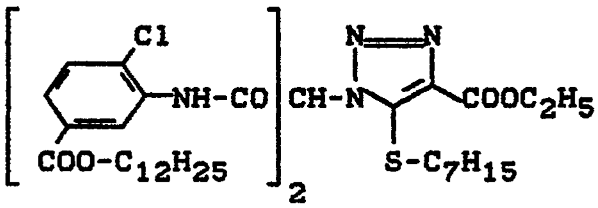

K-34

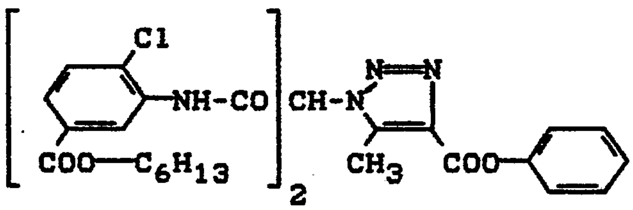

K-35

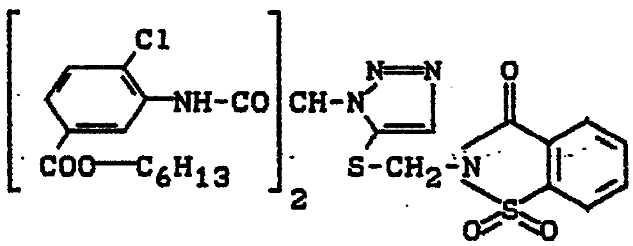

K-36

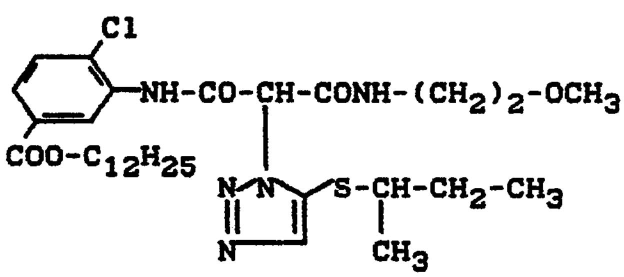

K-37

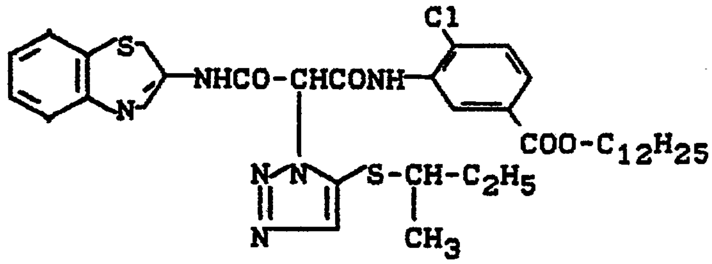

K-38

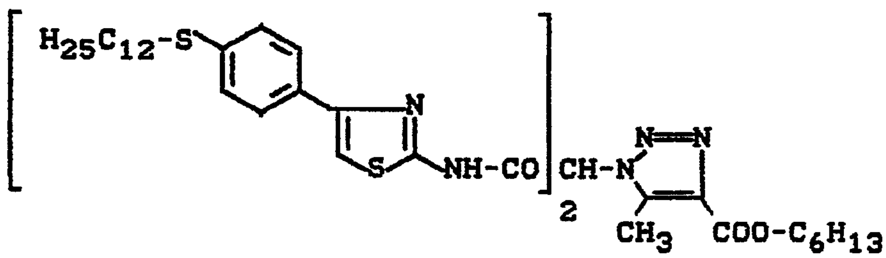

K-39

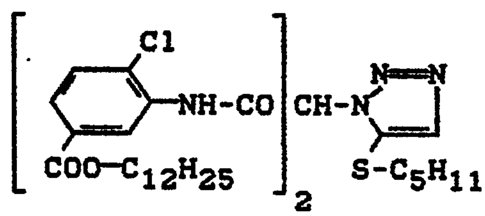

K-40

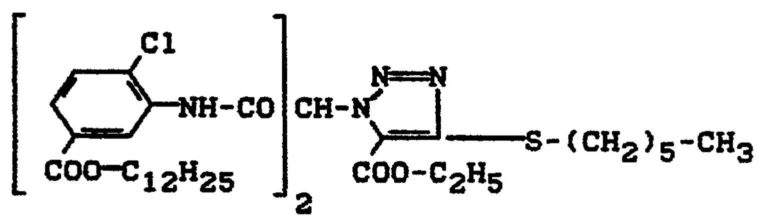

Die erfindungsgemäßen Kuppler der Formel I werden beispielsweise im Fall von Gelbkupplern leicht erhalten durch Kondensation der bekannten α-Halogenacylacetanilide der Formel V

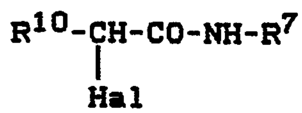

V

worin bedeuten
$R^7$ einen gegebenenfalls ein- oder mehrfach substituierten Arylrest;
$R^{10}$ einen Acylrest, z.B. einen Benzoylrest, einen Pivaloylrest oder einen Carbamoylrest;
Hal ein Halogenatom, insbesondere Chlor oder Brom,
mit Triazolen der Formel II.

Die Umsetzung wird dabei vorteilhaft in einem organischen Lösungsmittel wie Dimethylformamid, Acetonitril oder Aceton in Gegenwart einer Base wie Triethylamin oder Ätzkali durchgeführt.

Die Herstellung der Inhibitoren INH-1 bis INH-18 aus 4-Methyl-1H-1,2,3-triazol-5-carbonsäuremethylester (beschrieben in Klein et al., J. Heterocyclic Chem. 13, 589 (1976)) erfolgte durch Umesterung mit den entsprechenden Alkoholen unter Na-alkoholat-Katalyse bei 60 bis 100°C. Die Herstellung der Inhibitoren INH-19 bis INH-28, INH-37 bis INH-39 und INH-50 bis INH-55 erfolgt durch Umsetzung der

entsprechenden Triazolcarbonsäurechloride mit den Hydroxyarylverbindungen unter Pyridinzusatz in Toluol bei Raumtemperatur.

Die Verbindungen INH-29 bis INH-32 wurden durch Alkylierung der durch Na-alkoholat-katalysierten Kondensation von 4-Methyl-1H-1,2,3-triazol-5-carbonsäuremethylester mit Thiosemicarbazid erhaltenen SH-Verbindung hergestellt.

Die Herstellung von 4-(Alkyl)-mercapto-1H-1,2,3-triazol-5-carbonsäureestern, die zu den Verbindungen INH-44 bis INH-55 führen, ist beschrieben in Nemeryuk et al., Coll. Czech. Chem. Commun. 51, 215 (1981) und Goerdeler et al., Ber 99, 1618 (1966).

Die Herstellung der Verbindungen INH-56 bis INH-59 erfolgt durch Alkylierung von 4-Mercapto-1,2,3-triazol, z.B. im Fall der Verbindung INH-56 mit Butylbromid.

Da die Triazole der Formel II in verschiedenen tautomeren Formen auftreten können und dem entsprechenden Azeniation demgemäß verschiedene mesomere Grenzstrukturen zugeordnet werden können, ist bei der Kondensation die Verknöpfung mit dem C-Atom der Kupplungsstelle über verschiedene Ringstickstoffatome denkbar, so daß das Auftreten entsprechender Isomerer erklärbar ist. Diese Isomerie hat jedoch keinen Einfluß auf die Gebrauchseigenschaften der erfindungsgemäßen DIR-Kuppler, so daß sich ein Eingehen auf die Struktur der möglichen Isomeren erübrigt.

Die Herstellung der erfindungsgemäßen Kuppler wird im folgenden am Beispiel der Verbindungen K-2 und K-27 näher erläutert.

Herstellung der Verbindung K-2

10 g des chlorierten Kupplers

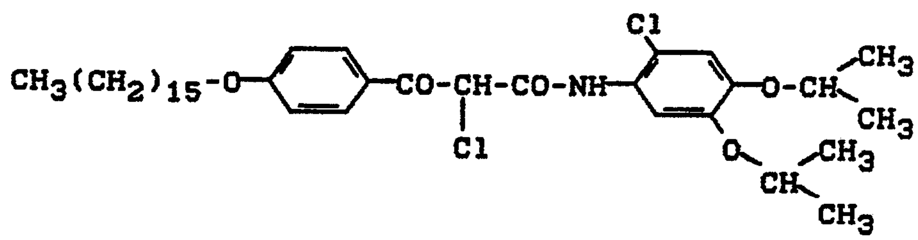

und 3 g 4-Methyl-1H-1,2,3-triazol-5-carbonsäurebutylester wurden zusammen mit 4,1 g Kaliumcarbonat in 50 ml Toluol 15 h gerührt. Es wurden 7 ml Eisessig zugegeben, 1 h gerührt, vom Rückstand abgesaugt und die Lösung eingeengt. Das Öl wurde an einer Kieselgelsäule mit einer Mischung Toluol:Essigester = 95:5 chromatographiert und die reinen Fraktionen eingeengt. Das so erhaltene Öl wurde in 60 ml Methanol über Nacht verrührt, die Kristalle abgesaugt, mit Methanol gewaschen und getrocknet. Ausbeute: 2,5 g Verbindung K-2 (DIR-Kuppler).

Herstellung der Verbindung K-27

Stufe 1: 4-Butylthio-1,2,3-triazol (INH-56)

31,8 (0,2 mol) 4-Mercapto-1,2,3-triazol-Natriumsalzdihydrat werden in 200 ml Methanol gelöst. Es werden 21,7 ml (0,2 mol) n-Butylbromid zugegeben und das Gemisch 2 h unter Rückfluß erhitzt. Danach wird auf Eiswasser aufgetragen und das sich abscheidende Öl in Methylenchlorid aufgenommen. Nach Abtrennen der organischen Phase, waschen mit Wasser und trocknen über Natriumsulfat wird das Lösungsmittel entfernt. Das rohe Produkt wird über Säulenchromatographie an Kieselgel mit Toluol/Essigester (20:2) gereinigt. Es wird ein klares Öl erhalten mit einer Ausbeute von 22,8 g (72% der Theorie).

Stufe 2: Verbindung K-27

4,27 g (30 mmol) des in Stufe 1 erhaltenen Produktes werden in 200 ml Essigester unter Zusatz von 5,75 g (50 mmol) Tetramethylguanidin vorgelegt. Bei Raumtemperatur werden unter Rühren 20,7 g α-Brommalonsäure-di-(2-chlor-5-dodecyloxycarbonyl)-anilid (25 mmol) portionsweise innerhalb 15 min zugegeben. Nach einer weiteren Stunde wird das Reaktionsgemisch zu einer Mischung von Eiswasser mit wenig Eisessig gegeben. Die organische Phase wird abgetrennt, mit Wasser gewaschen, über Natriumsulfat getrocknet und vom Lösungsmittel befreit.

Der erhaltene ölige Rückstand wird mit 20 ml einer 3:2-Mischung von Methanol mit Ethylacetat verrührt. Nach Kristallisation wird durch Filtration vom Lösungsmittelgemisch getrennt. Das erhaltene Produkt wird in 200 ml einer Mischung von Methanol und Acetonitril im Verhältnis 1:1 nochmals verrührt. Nach Abtrennen durch Filtration werden 6,9 g (31% d. Theorie) der Verbindung K-27 vom Schmelzpunkt 78-79°C erhalten.

Die Verbindungen der vorliegenden Erfindung eignen sich für die Verwendung als Kuppler in farbfotografischen, insbesondere mehrschichtigen Aufzeichnungsmaterialien. Als Gelbkuppler werden sie bevorzugt in oder zugeordnet zu einer lichtempfindlichen Silberhalogenidemulsionsschicht mit einer überwiegenden Empfindlichkeit für den blauen Spektralbereich des sichtbaren Lichtes verwendet. Der besondere Vorteil der erfindungsgemäßen Kuppler, nämlich eine vergleichsweise geringe Entwicklungsinhibierung in der Schicht, der eine solche Verbindung zugeordnet ist, neben einer vergleichsweise hohen Entwicklingsinhibierung in benachbarten nicht zugeordneten Schichten, kommt naturgemäß besonders dann zum Tragen, wenn es sich um ein mehrschichtiges farbfotografisches Aufzeichnungsmaterial handelt, das neben einer überwiegend blauempfindlichen Silberhalogenidemulsionsschicht weitere lichtempfindliche Silberhalogenidemulsionsschichten enthält mit überwiegender Empfindlichkeit für den grünen bzw. roten Spektralbereich des sichtbaren Lichtes. In entsprechender Weise werden die erfindungsgemäßen Kppler als Purpurkuppler bevorzugt einer grünempfindlichen Schicht bzw. als Blaugrünkuppler einer rotempfindlichen Schicht zugeordnet. Kuppler, die nur wenig Farbe bei der Entwicklung ergeben, können wahlweise einer blauempfindlichen, einer grünempfindlichen oder einer rotempfindlichen Schicht oder auch mehreren dieser Schichten zugeordnet werden, ohne daß eine Farbverfälschung zu befürchten ist.

Auch als Farbkuppler können die erfindungsgemäßen Kuppler wegen ihrer außerordentlich hohen Wirksamkeit in vergleichsweise geringen Mengen eingesetzt werden um die erwünschten Effekte, insbesondere die Interimageeffekte hervorzubringen. Dies ermöglicht es beispielsweise, einen erfindungsgemäßen Gelb-DIR-Kuppler nicht nur in den blauempfindlichen Gelbfarbstoff erzeugenden Schichten sondern auch in anderen Schichten einzusetzen, ohne daß dort eine zu hohe unerwünschte Nebendichte auftritt. Die erfindungsgemäßen DIR-Kuppler sind somit als Gelbkuppler mit Vorteil auch in Purpurschichten wie auch in Blaugrünschichten anwendbar. Entsprechendes gilt auch für die Purpurkuppler und die Blaugrünkuppler.

Bei der Herstellung des lichtempfindlichen farbfotografischen Aufzeichnungsmaterials können die diffusionsfesten DIR-Kuppler der vorliegenden Erfindung gegebenenfalls zusammen mit anderen Kupplern in bekannter Weise in die Gießlösung der Silberhalogenidemulsionsschichten oder anderer Kolloidschichten eingearbeitet werden. Beispielsweise können öllösliche oder hydrophobe Kuppler vorzugsweise aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Lösung des Kupplers braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Als lichtempfindliche Silberhalogenidemulsionen eignen sich Emulsionen von Silberchlorid, Silberbromid oder Gemischen davon, evtl. mit einem geringen Gehalt an Silberiodid bis zu 10 mol-% in einem der üblicherweise verwendeten hydrophilen Bindmittel. Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch und spektral sensibilisiert sein, und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sich aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Blaugrün, Purpur und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (z.B. Blaugrün, Purpur, Gelb) zugeordnet ist.

Jeder der unterschiedlich spektral sensibilisierten Silberhalogenidemulsionsschichten kann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, in der Regel ein Kuppler vom Phenol- oder α-Naphtholtyp. Vorteilhafte Blaugrünkuppler sind beispielsweise beschrieben in EP-A-0 028 099, EP-A-0 067 689, EP-A-0 175 573 und EP-A-0 184 057. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichungen "Farbkuppler" von W. PELZ im "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Acedemic Press (1971), verwiesen.

Bei den Farbkupplern kann es sich sowohl um übliche 4-Äquivalentkuppler handeln als auch um 2-Äquivalentkuppler, bei denen zur Farberzeugung eine geringere Menge Silber halogenid erforderlich ist. 2-Äquivalentkuppler leiten sich bekanntlich von den 4-Äquivaltenkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalenkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner die bekannten DIR-Kuppler zu rechnen, bei denen es sich um Kuppler handelt, die in der Kupplungsstelle einen abspaltbaren Rest enthalten, der bei Reaktion mit Farbentwickleroxidationsprodukten als diffundierender Entwicklungsinhibitor in Freiheit gesetzt wird. Auch andere fotografisch wirksame Verbindungen, z.B. Entwicklungsbeschleuniger oder Schleiermittel, können bei der Entwicklung aus solchen Kupplern freigesetzt werden.

Erfindungsgemäß enthält das farbfotografische Aufzeichnungsmaterial zusätzlich mindestens einen 2-Äquivalent gelbkuppler der Formel I, wobei dieser Kuppler nicht nur in der Gelbschicht, sondern auch in der Purpurschicht und/oder auch in der Blaugrünschicht sowie auch in einer zu einer der genannten Schichten benachbarten nicht lichtempfindlichen Schicht enthalten sein kann.

Über die genannten Bestandteile hinaus kann das farbfotografische Aufzeichnungsmaterial der vorliegenden Erfindung weitere Zusätze enthalten, zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es vorteilhaft, in einer oder mehreren der in dem Aufzeichnngsmaterial enthaltenen Schichten, vorzugsweise in einer der oberen Schichten, UV-absorbierende Verbindungen zu verwenden. Geeignete UV-Absorber sind beispielsweise in US-A-3 253 921, DE-C-2 036 719 und EP-A-0 057 160 beschrieben.

Für die erfindungsgemäßen Materialien können die üblichen Schichtträger verwendet werden, siehe Research Disclosure Nr. 17 643, Abschnitt XVII.

Als Schutzkolloid bzw. Bindemittel für die Schichten des Aufzeichnungsmaterials sind die üblichen hydrophilen filmbildenden Mittel geeignet, z.B. Protein, insbe sondere Gelatine. Begußhilfsmittel und Weichmacher können verwendet werden. Verwiesen wird auf die in der Research Disclosure Nr. 17 643 in Abschnitt IX, XI und XII angegebenen Verbindungen.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der DE-A-22 18 009 zu härten, um farbfotografische Materialien zu erzielen, die für eine Hochtemperaturverarbeitung geeignet sind. Ferner ist es möglich, die fotografischen Schichten mit Härtern der Diazin-, Triazin- oder 1,2-Dihydrochinolin-Reihe zu härten oder mit Härtern vom Vinylsulfon-Typ. Weitere geeignete Härtungsmittel sind aus den DE-A-24 39 551, DE-A-22 25 230, DE-A-23 17 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Zur Herstellung farbfotografischer Bilder wird das erfindungsgemäße farbfotografische Aufzeichnungsmaterial mit einer Farbentwicklerverbindung entwickelt. Als Farbentwicklerverbindung lassen sich sämtliche Entwicklerverbindungen verwenden, die die Fähigkeit besitzen, in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer. Chem. Soc. <u>73</u>, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

<u>Beispiel 1</u>

Ein farbfotografisches Aufzeichnungsmaterial für die Colornegativ-Entwicklung wurde hergestellt, indem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben. Alle Silberhalogenidemulsionen waren pro 100 g $AgNO_3$ mit 0,5 g 4-Hydroxy-6-methyl-1,3,3a,7-tetraazainden stabilisiert.

Schicht 1 (Antihaloschicht) Schwarzes kolloidales Silbersol mit
0,4 g Ag und
3 g Gelatine

Schicht 2 (Zwischenschicht) 0,5 g Gelatine mit
0,05 g Verbindung SC-1

Schicht 3 (1. rotsensibilisierte Schicht) rotsensibilisierte Silberbromidiodidemulsion (5 Mol-% Iodid; mittlerer Korndurchmesser 0,50 µm) aus 3,5 g $AgNO_3$, mit 1,5 g Gelatine, 0,6 g Kuppler C-1, 0,06 g Maskenkuppler MC-1 und DIR-Kuppler wie in Tabelle 1 angegeben.

Schicht 4 (2. rotsensibilisierte Schicht) rotsensibilisierte Silberbromidiodidemulsion

Schicht 6 (1. grünsensibilisierte Schicht) grünsensibilisierte Silberbromidiodidemulsion (5 Mol-% Iodid; mittlerer Korndurchmesser 0,50 µm) aus 2,5 g $AgNO_3$, mit
1,4 g Gelatine,
0,6 g Kuppler M-1
0,07 g Maskenkuppler MC-2 und DIR-Kuppler wie
in Tabelle 1 angegeben.

Schicht 7 (2. grünsensibilisierte Schicht) grünsensibilisierte Silberbromidiodidemulsion (2 Mol-% Iodid; mittlerer Korndurchmesser 1,3 µm) aus 2,2 g $AgNO_3$, mit
1,0 g Gelatine
0,15 g Kuppler M-1
0,03 g Maskenkuppler MC-2

Schicht 8 (Zwischenschicht) 0,34 g Gelatine mit
0,1 g Verbindung W-1

Schicht 9 (Gelbfilterschicht) gelbes kolloidales Silbersol mit
71 mg Ag,
0,5 g Gelatine und
0,1 g Verbindung W-1

Schicht 10 (1. blauempfindliche Schicht) Silberbromidiodidemulsion (5 Mol-% Iodid; mittlerer Korndurchmesser 0,5 µm) aus 0,7 g $AgNO_3$, mit
1,4 g Gelatine,
0,6 g Kuppler Y-1
und DIR-Kuppler wie in Tabelle 1 angegeben.

Schicht 11 (2. blauempfindliche Schicht) Silberbromidiodidemulsion (10 Mol-% Iodid; mittlerer Korndurchmesser 1,5 µm) aus 1,5 g $AgNO_3$, mit
0,7 g Gelatine,
0,15 g Kuppler Y-1

Schicht 12 (Zwischenschicht) 0,7 g Gelatine

Schicht 13 (Härtungsschicht) 0,24 g Gelatine mit
0,7 g Carbamoylpyridiniumsalz (CAS Reg. No. 65411-60-1)

Folgende Verbindungen wurden verwendet:

Verbindung SC-1

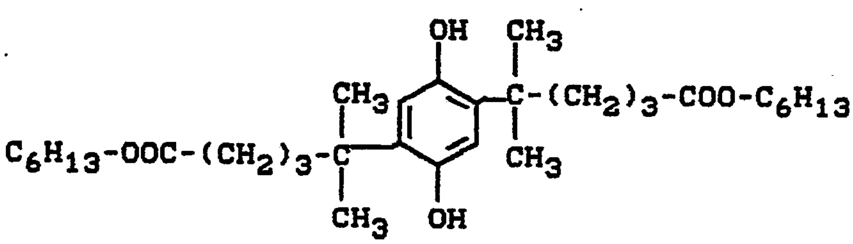

Kuppler C-1

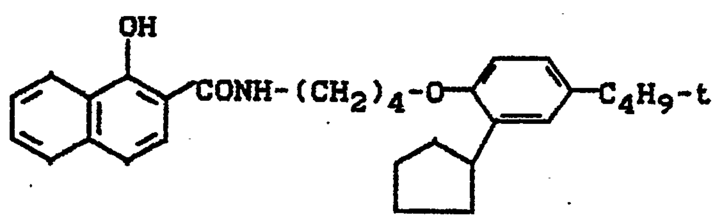

Verbindung W-1

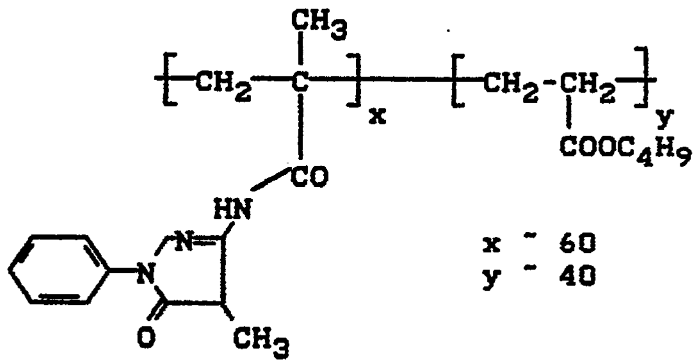


Kuppler M-1

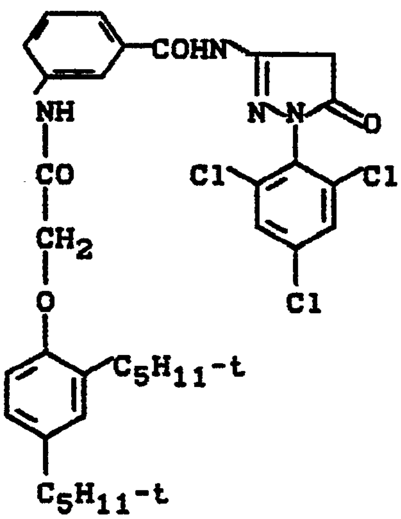

Maskenkuppler MC-1

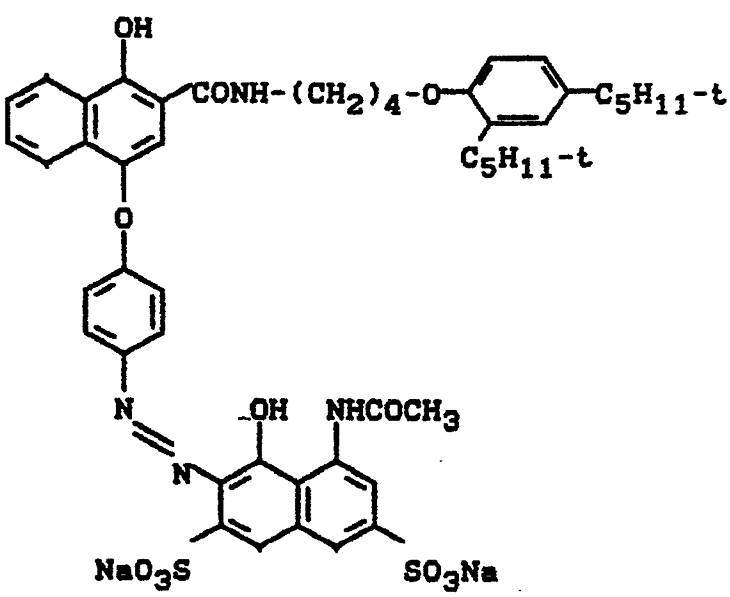

Maskenkuppler MC-2

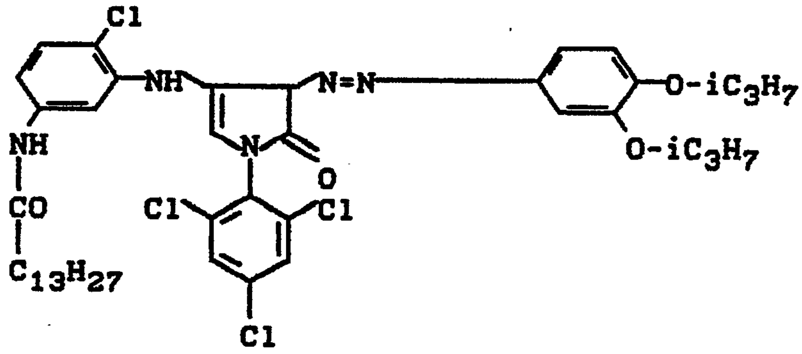

Kuppler Y-1

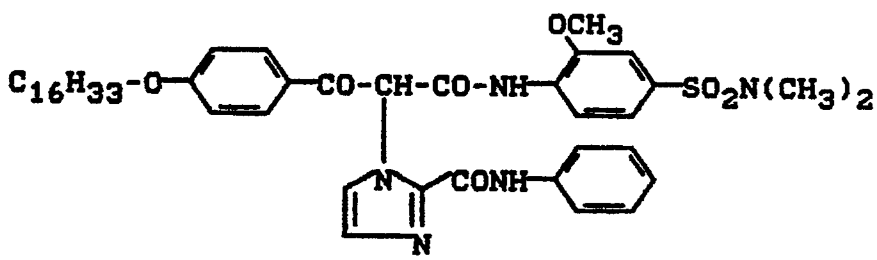

DIR-Kuppler A

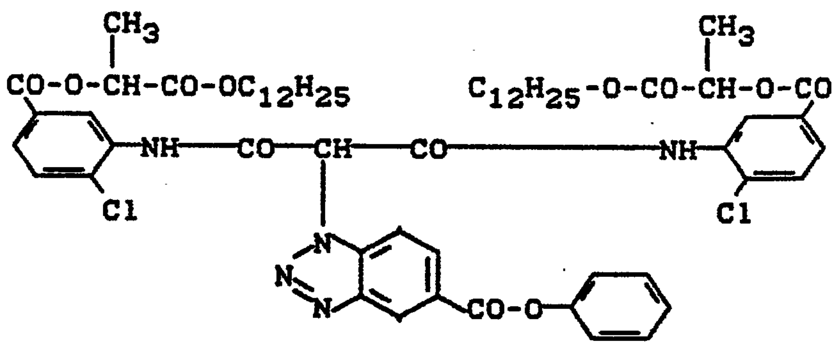

DIR-Kuppler A ist beschrieben als Verbindung Nr. 51 in DE-A-32 09 486)

DIR-Kuppler B

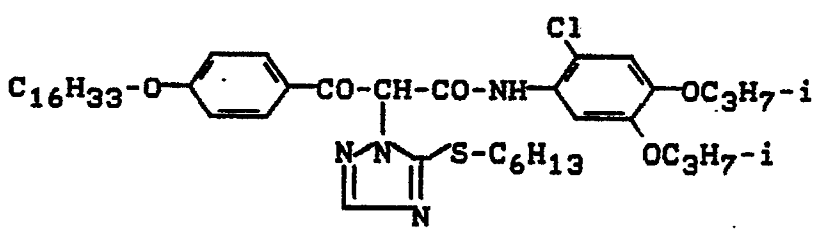

DIR-Kuppler B ist als Verbindung Nr. 3 beschrieben in der deutschen Patentanmeldung P 36 26 219.6)

DIR-Kuppler C

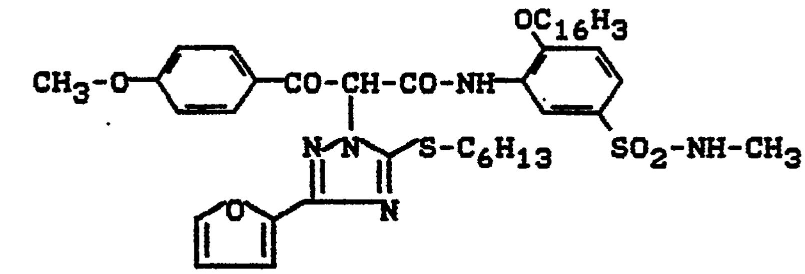

DIR-Kuppler C ist als Verbindung Nr. 4 beschrieben in DE-A-34 27 235.

Die Verbindungen C-1, M-1, MC-1, MC-2, Y-1 sowie die DIR-Kuppler wurden als Emulgate eingesetzt, wobei bezogen auf ein Teil der eingesetzten Verbindung 1 Teil Gelatine, 2 Teile Trikresylphosphat im Falle der Verbindungen M-1 und MC-2 bzw. Di-n-butylphthalat in allen anderen Fällen, und 0,1 Teile Na-Salz der Triisopropylnaphtalinsulfonsäure als Netzmittel verwendet wurden.

Von dem Aufzeichnungsmaterial des beschriebenen Aufbaus wurden verschiedene Versionen (Materialien 1-6) hergestellt, die sich ausschließlich durch die in den Schichten 3, 6 und 10 verwendeten DIR-Kuppler unterscheiden. Die Entwicklung wurde nach Aufbelichtung eines Graukeils durchgeführt wie beschrieben in "The Journal of Photographie", 1974, Seiten 597 und 598.

Die Ergebnisse nach Verarbeitung sind in Tabelle 1 dargestellt. Die Interimageeffekte IIE berechnen sich wie folgt:

$$IIE_{bg} = \frac{\gamma_{rot} - \gamma_w}{\gamma_w} \quad ; \quad IIE_{pp} = \frac{\gamma_{grün} - \gamma_w}{\gamma_w}$$

Dabei bedeutet:

$\psi_{rot}$ Gradation bei selekiver Belichtung mit rotem Licht

$\psi_{grün}$ Gradation bei selektiver Belichtung mit grünem Licht

$\psi_w$ Gradation bei Belichtung mit weißem Licht

Der in Tabelle 1 angegebene Kanteneffekt KE ist die Differenz zwischen Mikro- und Makrodichte bei Makrodicht = 1, wie beschrieben in James, The Theory of the Photografic Process, 5th Edition, Macmillan Publishing Co, Inc. 1977, Seite 611, Dabei bedeutet.

$KE_{bg}$ KE in der rotsensibilisierten Schicht

$KE_{pp}$ KE in der grünsensibilisierten Schicht

Tabelle 1

| Material | DIR-Kuppler Schicht 3 | DIR-Kuppler Schicht 6 | DIR-Kuppler Schicht 10 | $IIE_{bg}$ | $IIE_{pp}$ | $KE_{bg}$ | $KE_{pp}$ |
|---|---|---|---|---|---|---|---|
| 1 | $3{,}5\cdot10^{-5}$ Mol A | $1{,}8\cdot10^{-5}$ Mol A | $5{,}1\cdot10^{-4}$ Mol C | 45 | 55 | 0,37 | 0,27 |
| 2 | $5{,}1\cdot10^{-5}$ Mol B | $4{,}6\cdot10^{-5}$ Mol B | $5{,}1\cdot10^{-4}$ Mol C | 60 | 55 | 0,46 | 0,35 |
| 3 | $5{,}1\cdot10^{-5}$ Mol K-10 | $4{,}6\cdot10^{-5}$ Mol B | $5{,}1\cdot10^{-4}$ Mol C | 70 | 50 | 0,51 | 0,36 |
| 4 | $5{,}1\cdot10^{-5}$ Mol K-22 | $4{,}6\cdot10^{-5}$ Mol B | $5{,}1\cdot10^{-4}$ Mol C | 60 | 65 | 0,49 | 0,42 |
| 5 | $5{,}1\cdot10^{-5}$ Mol K-24 | $4{,}6\cdot10^{-5}$ Mol B | $5{,}1\cdot10^{-4}$ Mol C | 45 | 60 | 0,42 | 0,35 |
| 6 | $5{,}1\cdot10^{-5}$ Mol K-9 | $4{,}6\cdot10^{-5}$ Mol B | $5{,}1\cdot10^{-4}$ Mol C | 45 | 60 | 0,43 | 0,40 |
| 7 | $5{,}1\cdot10^{-5}$ Mol K-25 | $4{,}6\cdot10^{-5}$ Mol B | $5{,}1\cdot10^{-4}$ Mol C | 45 | 55 | 0,48 | 0,37 |
| 8 | $5{,}1\cdot10^{-5}$ Mol K-26 | $4{,}6\cdot10^{-5}$ Mol B | $5{,}1\cdot10^{-4}$ Mol C | 80 | 70 | 0,57 | 0,36 |
| 9 | $5{,}1\cdot10^{-5}$ Mol K-27 | $4{,}6\cdot10^{-5}$ Mol K-27 | $5{,}1\cdot10^{-4}$ Mol K-27 | 65 | 75 | 0,61 | 0,50 |
| 10 | $5{,}1\cdot10^{-5}$ Mol K-31 | $4{,}6\cdot10^{-5}$ Mol K-31 | $5{,}1\cdot10^{-4}$ Mol C | 75 | 45 | 0,65 | 0,45 |
| 11 | $5{,}1\cdot10^{-5}$ Mol K-39 | $4{,}6\cdot10^{-5}$ Mol K-39 | $5{,}1\cdot10^{-4}$ Mol C | 96 | 83 | 0,76 | 0,53 |
| 12 | $5{,}1\cdot10^{-5}$ Mol K-40 | $4{,}6\cdot10^{-5}$ Mol B | $5{,}1\cdot10^{-4}$ Mol C | 60 | 60 | 0,52 | 0,41 |

Besonders hohe Interimageeffekte und Kanteneffekte werden mit den erfindungsgemäßen DIR-Kupplern erhalten, wenn sie in mehr als einer Schicht verwendet werden. Auch dann sind die Vorteile der erfindungsgemäßen DIR-Kuppler verglichen mit solchen des Standes der Technik deutlich erkennbar.

**Patentansprüche**

1. Farbfotografisches Aufzeichnungsmaterial mit mindestens einer lichtempfindlichen Silberhalogenidemulsionsschicht und einem dieser zugeordneten Kuppler, der an seine Kupplungsstelle gebunden einen abspaltbaren 1,2,3-Triazolylrest trägt, dadurch gekennzeichnet, daß der Kuppler der folgenden Formel entspricht

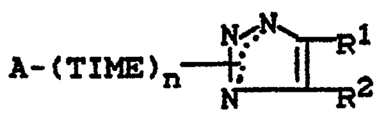

I

worin bedeuten
A den Rest eines Kupplers, der unter den Bedingungen der fotografischen Entwicklung als Folge der Reaktion mit dem Oxidationsprodukt eines Silberhalogenidentwicklungsmittels den Rest der Formel

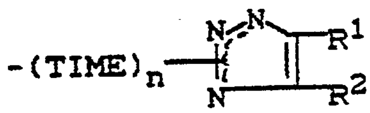

freisetzt;
TIME ein Bindeglied, das bei Reaktion des Kupplers mit dem Oxidationsprodukt eines Silberhalogenidentwicklungsmittels zusammen mit dem daran gebundenen Triazolring freigesetzt wird und seinerseits unter den Entwicklungsbedingungen den Triazolring verzögert freisetzt;
n 0,1 oder 2;
$R^1$ H, Alkyl, Aryl, $-S-R^3$, $-O-R^3$, $-CO-R^3$, eine Carbonsäureestergruppe, eine gegebenenfalls substituierte Aminogruppe, $-CONR^4-R^5$ oder eine heterocyclische Gruppe:
$R^2$ Halogen, -OH, Alkyl, eine Phenylgruppe, die substituiert ist mit mindestens einem der folgenden Reste:
Halogen; Alkyl; Aryl; eine gegebenenfalls substituierte Aminogruppe; Alkoxy; Aryloxy; Alkylthio; Arylthio; Nitro; Cyano; $-CF_3$ oder Acyl;
Naphthyl, $-S-R^3$, $-O-R^3$, $-CO-R^3$, eine Carbonsäureestergruppe mit mindestens 3 C-Atomen, eine gegebenenfalls substituierte Aminogruppe, $-CONR^4$, Cyano oder eine heterocyclische Gruppe;
$R^3$ Alkyl, Cycloalkyl, Aralkyl, Aryl, Alkenyl oder Alkinyl;
$R^4$ Alkyl, Aralkyl, Aryl, Acyl, $-NH^2$ oder Acylamino;
$R^5$ Wasserstoff, einen Rest wie $R^4$ oder $R^4$ und $R^5$ zusammen den erforderlichen Rest zur Vervollständigung einer cyclischen Aminogruppe.

2. Aufzeichnungsmaterial nach Anspruch 1, dadurch gekennzeichnet, daß A in Formel I den Rest eines phenolischen oder naphtholischen Blaugrünkupplers, eines Purpurkupplers oder eines Gelbkupplers bedeutet.

3. Aufzeichnungsmaterial nach Anspruch 2, dadurch gekennzeichnet, daß A in Formel I der folgenden Formel entspricht

$$R^{10}-\underset{|}{CH}-CO-NH-R^7$$

worin bedeuten,
$R^7$ einen gegebenenfalls ein- oder mehrfach substituierten Arylrest;
$R^{10}$ einen Acylrest.

4. Aufzeichnungsmaterial nach Anspruch 3, dadurch gekennzeichnet, daß A in Formel I der folgenden Formel entspricht

$$R^6-NH-CO-\underset{|}{CH}-CO-NH-R^7$$

worin $R^6$ eine heterocyclisch oder carbocyclisch aromatische Gruppe und $R^7$ Alkyl oder einen Rest wie $R^6$ bedeutet.

5. Aufzeichnungsmaterial nach einem der Ansprüche 1 bis 4, dadurch gekennzeichnet, daß in Formel I mindestens einer der Reste $R^1$ und $R^2$ für $-S-R^3$, $-COOR^8$ oder eine heterocyclische Gruppe steht, worin $R^3$ für Alkyl, Cycloalkyl, Aralkyl, Alkenyl oder Alkinyl, und $R^8$ für Alkyl mit 2 bis 10 C-Atomen oder Aryl steht.

6. Aufzeichnungsmaterial nach einem der Ansprüche 1 bis 5, dadurch gekennzeichnet, daß der Kuppler einer der beiden Formeln III und IV entspricht

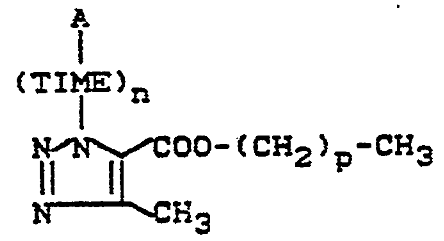

(III)

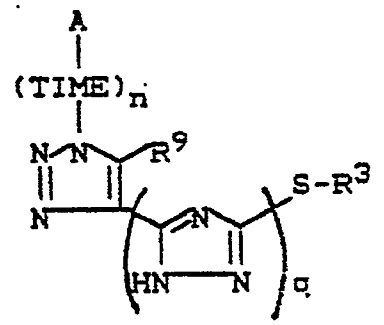

(IV)

worin TIME die in Anspruch 1 angegebene Bedeutung hat und worin bedeuten
A den Rest eines Kupplers;
$R^3$ Alkyl mit 1 bis 7 C-Atomen;
$R^9$ H, $-CH_3$, $-COO-(CH_2)_p-CH^3$;
n 0 oder 1;
p eine ganze Zahl von 1 bis 8;
q 0 oder 1.

7. Aufzeichnungsmaterial nach Anspruch 6, dadurch gekennzeichnet, daß der Kuppler ein Gelbkuppler ist und in einer überwiegend blauempfindlichen Silberhalogenidemulsionsschicht enthalten ist, und daß das Aufzeichnungsmaterial mindestens eine weitere überwiegend grünempfindliche oder überwiegend rotempfindliche Silberhalogenidemulsionsschicht enthält.

8. Farbfotografisches Aufzeichnungsmaterial nach Anspruch 6, dadurch gekennzeichnet, daß der Kuppler ein Gelbkuppler ist und in einer überwiegend rotempfindlichen Silberhalogenidemulsionsschicht enthalten ist.

9. Farbfotografisches Aufzeichnungsmaterial mit mindestens einer überwiegend blauempfindlichen Silberhalogenidemulsionsschichteneinheit, der mindestens ein Gelbkuppler zugeordnet ist, einer überwiegend grünempfindlichen Silberhalogenidemulsionsschichteneinheit, der mindestens ein Purpurkuppler zugeordnet ist, und einer überwiegend rotempfindlichen Silberhalogenidemulsionsschichteneinheit, der mindestens ein Blaugrünkuppler zugeordnet ist, dadurch gekennzeichnet, daß mindestens eine Teilschicht der überwiegend grünempfindlichen Silberhalogenidemulsionsschichteneinheit oder der überwiegend rotempfindlichen Silberhalogenidemulsionsschichteneinheit eine Verbindung einer der folgenden Formeln III und IV enthält:

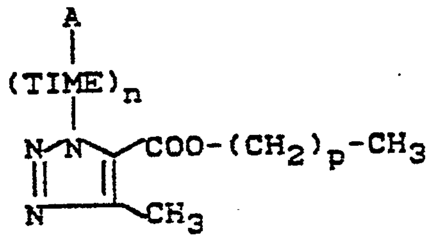

(III)

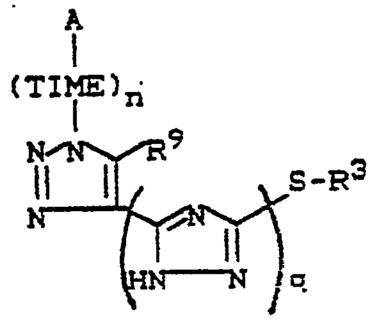

(IV)

worin TIME die in Anspruch 1 angegebene Bedeutung hat und worin bedeuten
A den Rest eines Gelbkupplers;
$R^3$ Alkyl mit 1 bis 7 C-Atomen;
$R^9$ H, $-CH_3$, $-COO-(CH_2)_p-CH_3$;
n 0 oder 1:
p eine ganze Zahl von 1 bis 8;
q 0 oder 1.

**Claims**

1. A colour photographic-recording material comprising at least one photosensitive silver halide emulsion layer and, associated therewith, a coupler which contains a releasable 1,2,3-trizolyl radical attached to its coupling position, characterized in that the coupler corresponds to the following formula

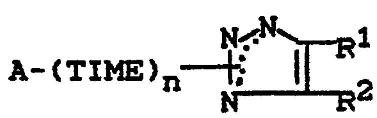

I

in which
A represents the residue of a coupler which couples with the oxidation product of a silver halide developer under the photographic development conditions and, in doing so, releases a radical corresponding to the following formula

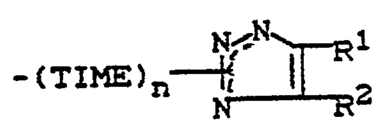

TIME represents a bond which, on reaction of the coupler with the oxidation product of a silver halide developer, is released together with the triazole ring attached thereto and, in turn, releases the triazole ring with delay under the development conditions;
n = 0, 1 or 2;
$R^1$ represents H, alkyl, aryl $-S-R^3$, $-O-R^3$, $-CO-R^3$, a carboxylic acid ester group, an optionally substituted amino group, $-CONR^4-R^5$ or a heterocyclic group;
$R^2$ represents halogen, -OH, alkyl, a phenyl group substituted by at least one of the following radicals: halogen; alkyl; aryl; an optionally substituted amino group; alkoxy; aryloxy; alkylthio; arylthio; nitro; cyano; $-CF_3$ or acyl;
napthyl, $-S-R^3$, $-O-R^3$, $-CO-R^3$, a carboxylic ester group containing at least 3 C atoms, an optionally substituted amino group, $-CONR^4$, cyano or a heterocyclic group:

$R^3$ represents alkyl, cycloalkyl, aralkyl, aryl, alkenyl or alkynyl;
$R^4$ represents alkyl, aralkyl, aryl, acyl, $-NH_2$ or acylamino;
$R^5$ represents hydrogen, a radical such as $R^4$ or $R^4$ and $R^5$ together represent the radical required to complete a cyclic amino group.

2. A recording material as claimed in claim 1, characterized in that A in formula I represents the residue of a phenolic or naphtholic cyan coupler, a magenta coupler or a yellow coupler.

3. A recording material as claimed in claim 2, characterized in that A in formula I corresponds to the following formula

$$R^{10}-\underset{|}{CH}-CO-NH-R^7$$

in which
$R^7$ is an optionally mono- or polysubstituted aryl radical;
$R^{10}$ represents an acyl radical.

4. A recording material as claimed in claim 3, characterized in that A in formula I corresponds to the following formula

$$R^6-NH-CO-\underset{|}{CH}-CO-NH-R^7$$

in which
$R^6$ is a heterocyclic or carbocyclic aromatic group and $R^7$ is alkyl or has the same meaning as $R^5$.

5. A recording material as claimed in any of claims 1 to 4, characterized in that, in formula I, at least one of the substituents $R^1$ and $R^2$ is $-S-R^3$, $COOR^8$ or a heterocyclic group, where $R^3$ is alkyl, cycloalkyl, aralkyl, alkenyl or alkynyl and $R^8$ is alkyl containing 2 to 10 C atoms or aryl.

6. A recording material as claimed in any of claims 1 to 5, characterized in that the coupler corresponds to one of formulae III and IV

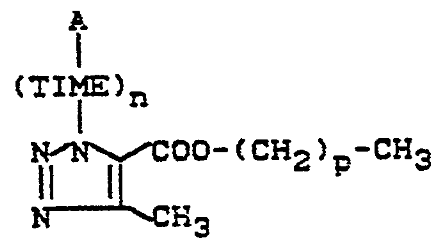

(III)

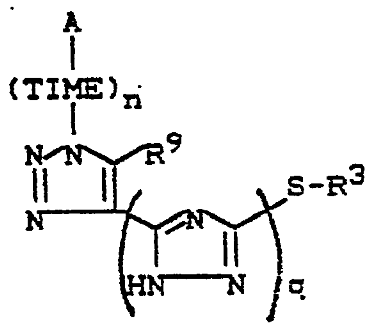

(IV)

in which
TIME is as defined in claim 1 and
A is the residue of a coupler,
$R^3$ is $C_{1-7}$ alkyl,
$R^9$ H, $-CH_3$, $-COO-(CH_2)_p-CH^3$;
n is 0 or 1;
p is an integer of 1 to 8;
q is 0 or 1.

7. A recording material as claimed in claim 6, characterized in that the coupler is a yellow coupler and is contained a predominantly blue-sensitive silver halide emulsion layer and in that the recording material

contains at least one other predominantly green-sensitive or predominantly red-sensitive silver halide emulsion layer.

8. A colour photographic recording material as claimed in claim 6, characterized in that the coupler is a yellow coupler and is contained in a predominantly red-sensitive silver halide emulsion layer.

9. A colour photographic recording material comprising at least one predominantly blue-sensitive silver halide emulsion layer unit with which at least one yellow coupler is associated, a predominantly green-sensitive silver halide emulsion layer unit with which at least one magenta coupler is associated and a predominantly red-sensitive silver halide emulsion layer unit with which at least one cyan coupler is associated, characterized in that at least one partial layer of the predominantly green-sensitive silver halide emulsion layer unit or of the predominantly red-sensitive silver halide emulsion layer unit contains a compound corresponding to one of formula III and IV below:

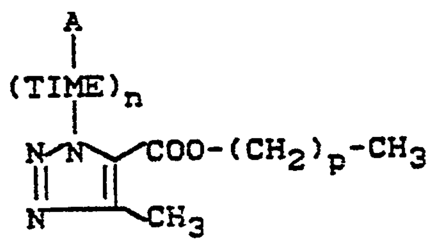

(III)

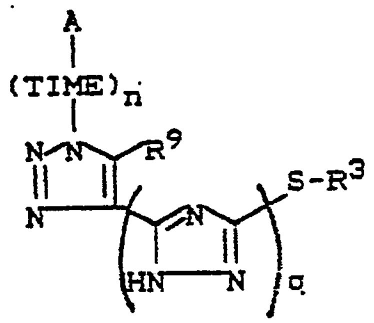

(IV)

in which
TIME is as defined in claim 1 and
A is the residue of a coupler,
$R^3$ is $C_{1-7}$ alkyl,
$R^9$ is H, $-CH_3$, $-COO-(CH_2)_p-CH_3$;
n is 0 or 1;
p is an integer of 1 to 8;
q is 0 or 1.

**Revendications**

1. Matériau d'enregistrement pour la photographie en couleurs comprenant au moins une couche photosensible d'émulsion d'halogénure d'argent et un coupleur associé à cette couche, ce coupleur portant, fixé sur son site de couplage, un groupe 1,2,3-triazolyle scindable, caractérisé en ce que le coupleur répond à la formule

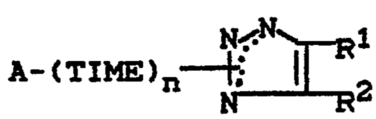

I

dans laquelle A est le reste d'un coupleur qui, dans les conditions du développement photographique, à la suite de la réaction avec le produit d'oxydation d'un révélateur de l'halogénure d'argent, libère le groupe de formule

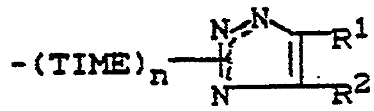

TIME est un chaînon de liaison, libéré avec le cycle triazole auquel il est relié à la réaction du coupleur avec le produit d'oxydation d'un révélateur de l'halogénure d'argent et qui libère lui-même avec retard, dans les conditions du développement, le cycle triazole,

n est égal à 0, 1 ou 2;

$R^1$ représente H, un groupe alkyle, aryle, $-S-R^3$, $-O-R^3$, $-CO-R^3$, un groupe ester d'acide carboxylique, un groupe amino éventuellement substitué, un groupe $-CONR^4-R^5$ ou un groupe hétérocyclique;

$R^2$ représente un halogène, un groupe -OH, alkyle, un groupe phényle portant au moins un des substituants suivants: les halogènes; les groupes alkyle; les groupes aryle; un groupe amino éventuellement substitué; les groupes alcoxy; aryloxy; alkylthio; arylthio; nitro cyano; $-CF_3$ ou acyle;

un groupe naphtyle, $-S-R^3$, $-O-^3$, $-CO-R^3$, un groupe ester d'acide carboxylique à au moins 3 atomes de carbone, un groupe amino éventuellement substitué, un groupe $-CONR^4$, un groupe cyano ou un groupe hétérocyclique;

$R^3$ représente un groupe alkyle, cycloalkyle, aralkyle, aryle, alcényle ou alcynyle;

$R^4$ représente un groupe alkyle, aralkyle, aryle, acyle, $-NH_2$ ou acylamino;

$R^5$ représente l'hydrogène, un substituant tel que ceux représentés par $R^4$, ou bien $R^4$ et $R^5$ forment ensemble le groupe nécessaire pour compléter un groupe amino cyclique.

2. Matériau d'enregistrement selon la revendication 1, caractérisé en ce que A représente, dans la formule I, le reste d'un coupleur phénolique ou naphtolique en bleu-vert, d'un coupleur en pourpre ou d'un coupleur en jaune.

3. Matériau d'enregistrement selon la revendication 2, caractérisé en ce que A, dans la formule I, répond à la formule suivante;

$$R^{10}-CH-CO-NH-R'$$

dans laquelle

$R^7$ représente un groupe aryle éventuellement mono- ou poly-substitué;

$R^{10}$ représente un groupe acyle.

4. Matériau d'enregistrement selon la revendication 3, caractérisé en ce que A, dans la formule I, répond à la formule suivante:

$$R^6-NH-CO-CH-CO-NH-R^7$$

dans laquelle $R^6$ représente un groupe aromatique hétérocyclique ou carboxylique et $R^7$ représente un groupe alkyle ou un substituant tel que ceux représentés par $R^6$.

5. Matériau d'enregistrement selon l'une des revendications 1 à 4, caractérisé en ce que, dans la formule I, au moins un des symboles $R^1$ et $R^2$ représente $-S-R^3$, $-COOR^8$ ou un groupe hétérocyclique, $R^3$ représentant un groupe alkyle, cycloalkyle, aralkyle, alcényle ou alcynyle et $R^8$ représentant un groupe alkyle en $C_2-C_{10}$ ou aryle.

6. Matériau d'enregistrement selon l'une des revendications 1 à 5, caractérisé en ce que le coupleur répond à l'une des deux formules III et IV

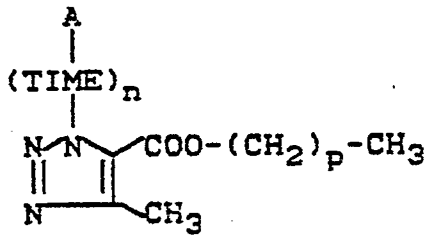

(III)

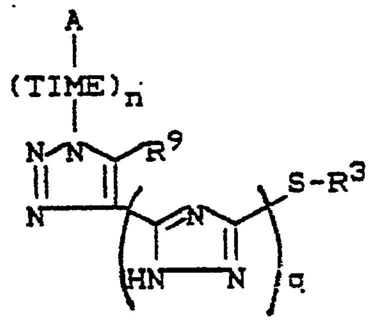

(IV)

dans lesquelles
TIME a les significations indiquées dans la revendication 1 et A est le reste d'un coupleur;
$R^3$ représente un groupe alkyle en $C_1$–$C_7$;
$R^9$ représente H, $-CH_3$, $COO-(CH_2)_p-CH_3$;
n est égal à 0 ou 1;
p est un nombre entier allant de 1 à 8;
q est égal à 0 ou 1.

7. Matériau d'enregistrement selon la revendication 6, caractérisé en ce que le coupleur est un coupleur jaune et est contenu dans une couche d'émulsion d'halogénure d'argent principalement sensible au bleu, et en ce que le matériau d'enregistrement contient au moins une autre couche d'émulsion d'halogénure d'argent principalement sensible au vert ou principalement sensible au rouge.

8. Matériau d'enregistrement pour la photographie en couleurs selon la revendication 6, caractérisé en ce que le coupleur est un coupleur jaune et est contenu dans une couche d'émulsion d'halogénure d'argent principalement sensible au rouge.

9. Matériau d'enregistrement pour la photographie en couleurs contenant au moins une unité de couches d'émulsion d'halogénure d'argent principalement sensible au bleu associée à au moins un coupleur jaune, une unité de couches d'émulsion d'halogénure d'argent principalement sensible au vert, associée à au moins un coupleur pourpre, et une unité de couches d'émulsion d'halogénure d'argent principalement sensible au rouge, associée à au moins une couche partielle de l'unité de couches d'émulsion d'halogénure d'argent principalement sensible au vert ou de l'unité de couches d'émulsion d'halogénure d'argent principalement sensible au rouge contient un composé répondant à l'une des formules III et IV ci-après:

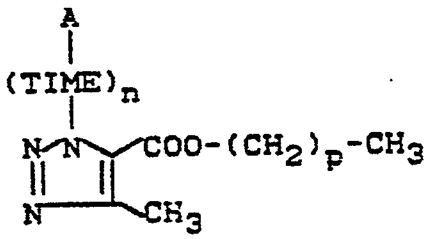

(III)

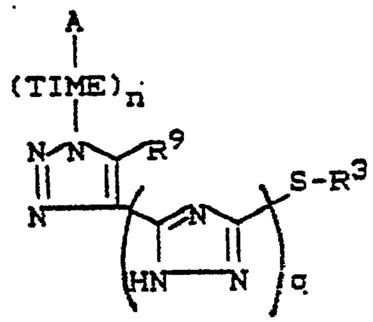

(IV)

dans lesquelles
TIME a les significations indiquées dans la revendication 1 et
A représente le reste d'un coupleur jaune;
$R^3$ représente un groupe alkyle en $C_1$–$C_7$;
$R^9$ représente H, $-CH_3$, $-COO-(CH_2)_p-CH_3$;
n est égal à 0 ou 1;
p est un nombre entier allant de 1 à 8;
q est égal à 0 ou 1.